# EUROPEAN PATENT SPECIFICATION

(11) **EP 0 855 390 B1**
(45) Date of publication and mention of the grant of the patent: **04.10.2001**
(21) Application number: 98400146.1
(22) Date of filing: 23.01.1998
(51) Int. Cl.: C07D 207/08, C07D 207/09

(54) **A process for producing optically active pyrrolidine derivatives**
Verfahren zur Herstellung optisch aktiver Pyrrolidine
Procédé pour la préparation de dérivés de pyrrolidine optiquement actifs

(30) Priority: 24.01.1997 JP 2451497
(43) Date of publication of application: 29.07.1998
(73) Proprietor: Takasago International Corporation, Tokyo 108 (JP)
(72) Inventor: Yuwasa, Yosifumi, Hiratsuka-shi, Kanagawa-ken (JP); Sotoguchi, Tsukasa, Hiratsuka-shi, Kanagawa-ken (JP); Seido, Nobuo, Hiratsuka-shi, Kanagawa-ken (JP)
(74) Representative: Gillard, Marie-Louise

(56) References cited:
- EP-A- 0 295 890
- WO-A-94/26708
- CHEMICAL ABSTRACTS, vol. 121, no. 9, 29 August 1994 Columbus, Ohio, US; abstract no. 108503x, page 1044; XP002063630 & JP 00 672 999 A (FUJISAWA PHARM CO)
- SCHROEDER M C ET AL: "SYNTHESIS OF THE FOUR STEREOISOMERS OF SEVERAL 3-(1-AMINOETHYL)PYRROLIDINES. IMPORTANT INTERMEDIATES IN THE PREPARATION OF QUINOLONE ANTIBACTERIALS" JOURNAL OF HETEROCYCLIC CHEMISTRY, vol. 29, no. 6, October 1992, pages 1481-1498, XP000647515
- Y. KIMURA: "SYNTHESIS AND STRUCTURE-ACTIVITY RELATIONSHIPS OF 7-[3-1-AMINOCYCLOALKYL)PYRROLIDINYL]-QUINO LONE ANTIBACTERIALS" CHEMICAL PHARMACEUTICAL BULLETIN , vol. 42, no. 7, 1994, pages 1442-54, XP002063628
- J. S. PLUMMER ET AL.: "STEREOSELECTIVE MICHAEL ADDITIONS OF NITROMETHANE YIELDING 3R(1S N-SUBSTITUTED AMINOETHYL)PYRROLIDINES" TETRAHEDRON LETTERS, vol. 34, no. 47, 1993, pages 7529-32, XP002063629

## Description

### BACKGROUND OF THE INVENTION

### 1. Field of the Invention

The present invention relates to a process for producing 3R,1'S)-3-[(1'-N-methylamino)ethyl]pyrrolidine as an intermediate for synthesis of quinolone type anti-fungus agents such as norfloxacin, ciprofloxacin etc.

### 2. Description of the Prior Art

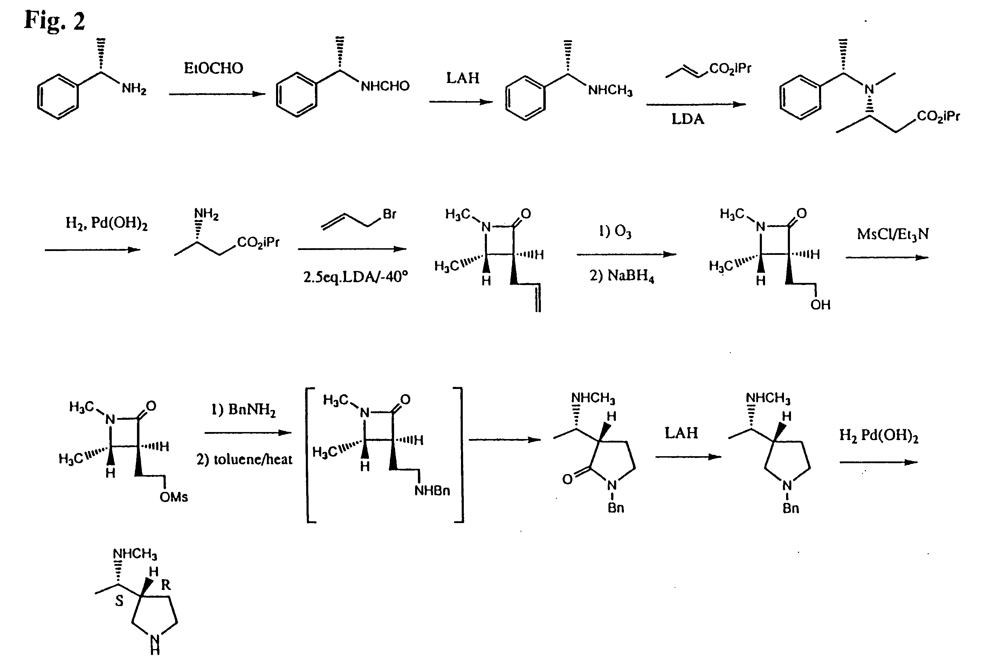

As shown in W09426708, a conventional process for producing (3R,1'S)-3-[(1'-N-methylamino)ethyl]pyrrolidine (14)involves subjecting optically active phenylethylamine as the starting material to formylation followed by the steps of monomethylation by reduction, addition reaction by crotonate, de-benzylation, conversion into β-lactam by allylation and ring closure, ozone oxidation, reduction with sodium borohydride, mesylation, reaction with benzylamine, formation of a pyrrolidinone ring by ring-enlarging reaction under heating and subsequent reduction with lithium aluminum hydride, and finally de-benzylation to give the final product of (3R,1'S)-3-[(1'-N-methylamino)ethyl]pyrrolidine (see FIG. 2).

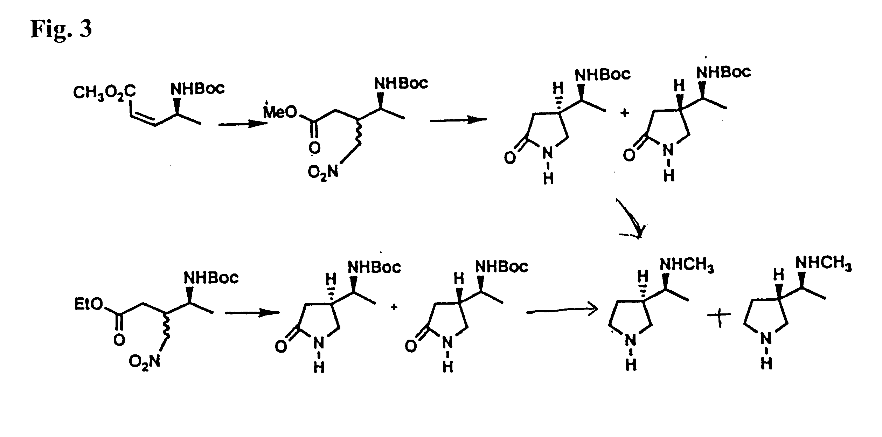

An alternative method, as shown in Tetrahedron Letters, 34:7529 (1993), involves subjecting L-alanine as the starting material to t-butoxycarbonylation (conversion into BOC) followed by the steps of conversion into β-ketoester by increasing carbon, reduction of the carbonyl group, mesylation, conversion into an unsaturated ester by 1,8-diazobicyclo[5,4,0]undeca-7-ene (DBU), addition of nitromethane, formation of a pyrrolidinone ring by reduction and ring closure with Raney nickel, and finally reduction with lithium aluminum hydride whereby (3R,1'S)-3-[(1'-N-methylamino)ethyl]pyrrolidine is obtained (see FIG. 3).

Further, a method of preparing racemates is known in which as shown in Chemical Pharmaceutical Bulletin Japan, 42: 1442 (1994) and Japanese Patent Application Laid-open Nos. 60-139830 and 279991/85, 4-carboxy-N-benzyl-2-pyrrolidone is acetylated and converted into an oxime, and the oxime is reduced and converted into BOC which is finally reduced with lithium aluminum hydride to give 3-[(1'-N-methylamino)ethyl]pyrrolidine.

These methods could not be said to be industrially adequate owing to many steps and complicated procedures and because expensive reagents and optically active materials are used therein.

### SUMMARY OF THE INVENTION

Therefore, the object of the present invention is to provide a production process in which optically active (3R,1'S)-3-[(1'-N-methylamino)ethyl] pyrrolidine used as an intermediate for synthesis of pharmaceutical preparations such as anti-fungus agents is produced by a safe and easy method.

As a result of their eager study in view of such circumstances, the present inventors established a production process which comprises subjecting 3-acetyl-1-benzyl-2-pyrrolidinone (1) to asymmetric hydrogenation by use of a complex formed from bidentate phosphine and ruthenium as a catalyst to produce (3S,1'R)-1-benzyl-3-[(1'-hydroxy)ethyl]-2-pyrrolidinone (2) of high purity in high yield, then reducing it with sodium borohydride and sulfuric acid or lithium aluminum hydride to give (3R,1'R)-1-benzyl-3-[(1'-hydroxy)ethyl]pyrrolidine (3), allowing the resulting (3R,1'R)-1-benzyl-3-[(1'-hydroxy)ethyl]pyrrolidine (3) to be treated with methanesulfonyl chloride or p-toluenesulfonyl chloride in the presence of a base to give (3R,1'R)-1-benzyl-3-[(1'-methanesulfonyloxy)ethyl]pyrrolidine (4a) or (3R,1'R)-1-benzyl-3-[(1'-p-toluenesulfonyloxy)ethyl]pyrrolidine (4b), followed by methylamination thereof with methylamine, whereby (3R,1'S)-1-benzyl-3-[(1'-N-methylamino)ethyl]pyrrolidine (5) could be obtained, and the resulting (3R,1'S)-1-benzyl-3-[(1'-N-methylamino)ethyl]pyrrolidine (5) was subjected to de-benzylation so that the desired (3R,1'S)-3-[(1'-N-methylamino)ethyl]pyrrolidine (14) could be produced in a safe and easy manner, and the present inventors thereby completed the present invention.

To attain the above-mentioned objective, there is provided a process for producing (3R,1'S)-1-benzyl-3-[(1'-N-methylamino)ethyl]pyrrolidine represented by formula (5): 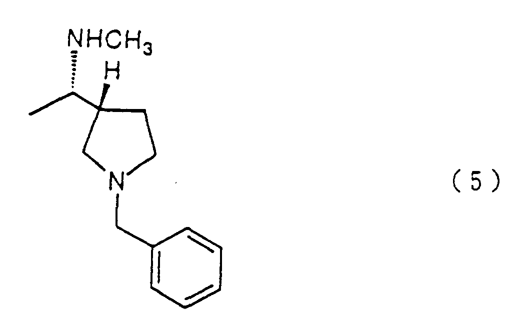 which comprises asymmetric hydrogenation of 3-acetyl-1-benzyl-2-pyrrolidinone represented by formula (1): 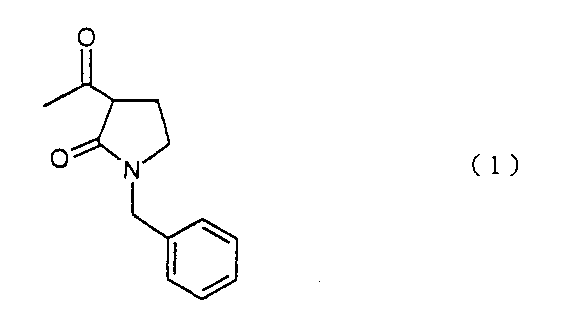 by use of a complex formed from bidentate phosphine and ruthenium as a catalyst to produce (3S,1'R)-1-benzyl-3-[(1'-hydroxy)ethyl]-2-pyrrolidinone represented by formula (2): 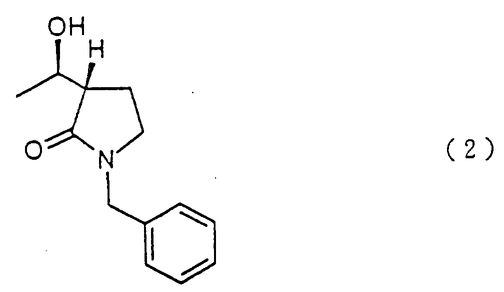 reducing the resulting (3R,1'R)-1-benzyl-3-[(1'-hydroxy)ethyl]-2-pyrrolidinone to produce (3R,1'R)-1-benzyl-3-[(1'-hydroxy)ethyl]pyrrolidine represented by formula (3): 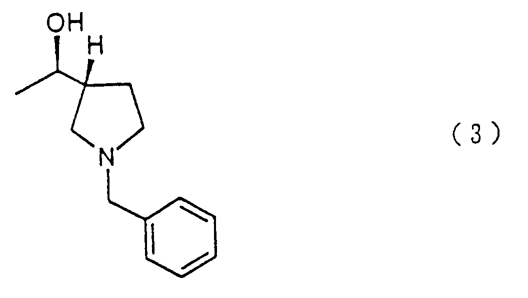 mesylating and/or tosylating the resulting (3R,1'R)-1-benzyl-3-[(1'-hydroxy)ethyl]pyrrolidine to produce (3R,1'R)-1-benzyl-3-[(1'-methanesulfonyloxy)ethyl]pyrrolidine and/or (3R,1'R)-1-benzyl-3-[(1'-p-toluenesulfonyloxy)ethyl]pyrrolidine represented by formula (4) : 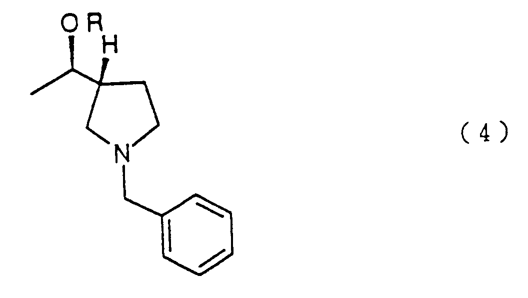 wherein R represents a methanesulfonyl group or p-toluenesulfonyl group, and methylaminating the resulting (3R,1'R)-1-benzyl-3-[(1'-methanesulfonyloxy)ethyl]pyrrolidine or (3R,1'R)-1-benzyl-3-[(1'-p-toluenesulfonyloxy)ethyl]pyrrolidine.

In the above process, the bidentate phosphine may be a bidentate phosphine represented by the following general formula (6) : 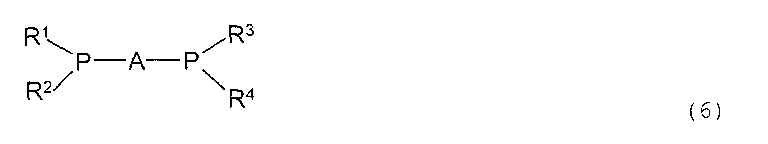 wherein R¹, R², R³ and R⁴ may be the same or different and each represent a phenyl group, cyclohexyl group, cycopentyl group, C₁ to C₄ lower alkyl group, C₁ to C₄ lower alkoxy group, or halogen-substituted phenyl group;
A is represented by the following general formula (7) : 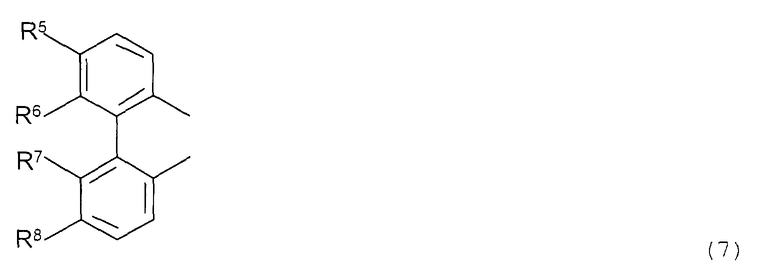 wherein R⁵ and R⁸ each represent a hydrogen atom while R⁶ and R⁷ each represent a methyl group, or R⁵ and R⁶ or R⁷ and R⁸ may be combined to form a tetramethylene group, or is represented by the following general formula (8) : 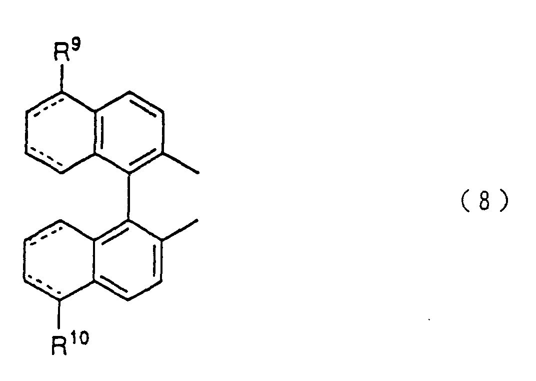 wherein R⁹ and R¹⁰ may be the same or different and each represent a hydrogen atom, amino group, acetylamino group, or -SO₃M whereupon M stands for a hydrogen atom or alkali metal atom, and the combination of a full line and a broken line signifies either a saturated bond or a double bond.

In the same process, the complex formed from bidentate phosphine and ruthenium may be a complex represented by the following general formula (9):

Ru ₓH_{y}Cl _{z}(L)₂(S)ₚ (9)

wherein L is a bidentate phosphine represented by the following general formula (6): 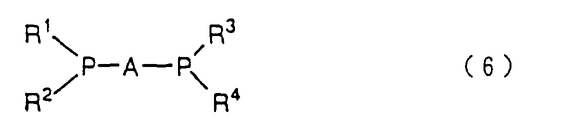 wherein R¹, R², R³, R⁴ and A have the same meanings as defined above, and S is represented by the following general formula (10):

NR¹¹R¹²R¹³ (10)

wherein R¹¹, R¹² and R¹³ may be the same or different and each represent a lower alkyl group, and when y is 0, x is 2, z is 4, and p is 1; when y is 1, x is 1, z is 1, and p is 0.

Further, the complex formed from bidentate phosphine and ruthenium may be a complex represented by the following general formula (11) :

Ru (OCOR¹⁴)₂(L) (11)

wherein R¹⁴ represents a C₁ to C₄ lower alkyl group and trifluoromethyl group, and L represents the same bidentate phosphine as defined above.

The complex formed from bidentate phosphine and ruthenium may also be a complex represented by the following general formula (12) :

Ru X₂ (L) (12)

wherein X represents a halogen atom, and L represents the same bidentate phosphine as defined above.

Still further, the complex formed from bidentate phosphine and ruthenium may be a complex represented by the following general formula (13) :

RuXₖJₘ (L) Qₙ (13)

wherein X represents a halogen atom, and J represents benzene, p-cymene, C₁ to C₄ lower alkyl benzoate or acetonitrile, Q represents a halogen atom, ClO₄, PF₆ and BF₄, L represents the same bidentate phosphine as defined above; in case J is benzene, p-cymene or lower alkyl benzoate, m is 1 and n is 1 when k is 1; and in case J is acetonitrile, m is 1 and n is 1 when k is 1, and m is 4 and n is 2 when k is 0.

In the above-mentioned process, (3S,1'R)-1-benzyl-3-[(1'-hydroxy)ethyl]-2-pyrrolidinone represented by formula (2) may be reduced with lithium aluminum hydride, borane, sodium borohydride and a mineral acid or with sodium borohydride and an organic acid.

Further in the same process,(3R,1'R)-1-benzyl-3-[(1'-hydroxy)ethyl]pyrrolidine represented by formula (3) may be reacted with methanesulfonyl chloride or p-toluenesulfonyl chloride in the presence of a base.

Then, in the above process, (3R,1'R)-1-benzyl-3-[(1'-methanesulfonyloxy)ethyl]pyrrolidine and/or (3R,1'R)-1-benzyl-3-[(1'-p- toluenesulfonyloxy)ethyl]pyrrolidine represented by formula (4) may be reacted with methylamine in an organic solvent.

In the above-mentioned processes, the product (4) used is a new intermediate product.

### BRIEF DESCRIPTION OF THE DRAWINGS

FIG. 1 shows a preferable process for preparing (3R,1'S)-1-benzyl-3-[(1'-N-methylamino)ethyl]pyrrolidine from 3-acethyl-1-benzyl-2-pyrrolidinone as the starting material.

FIG. 2 shows a conventional process for preparing (3R,1'S)-3-[(1'-N-methylamino)ethyl]pyrrolidine from optically active phenylethylamine as the starting material.

FIG. 3 a conventional process for preparing (3R,1'S)-3-[(1'-N-methylamino)ethyl]pyrrolidine from L-alanine as the starting material.

### PREFERRED EMBODIMENTS OF THE INVENTION

Hereinafter, the present invention is described in detail.

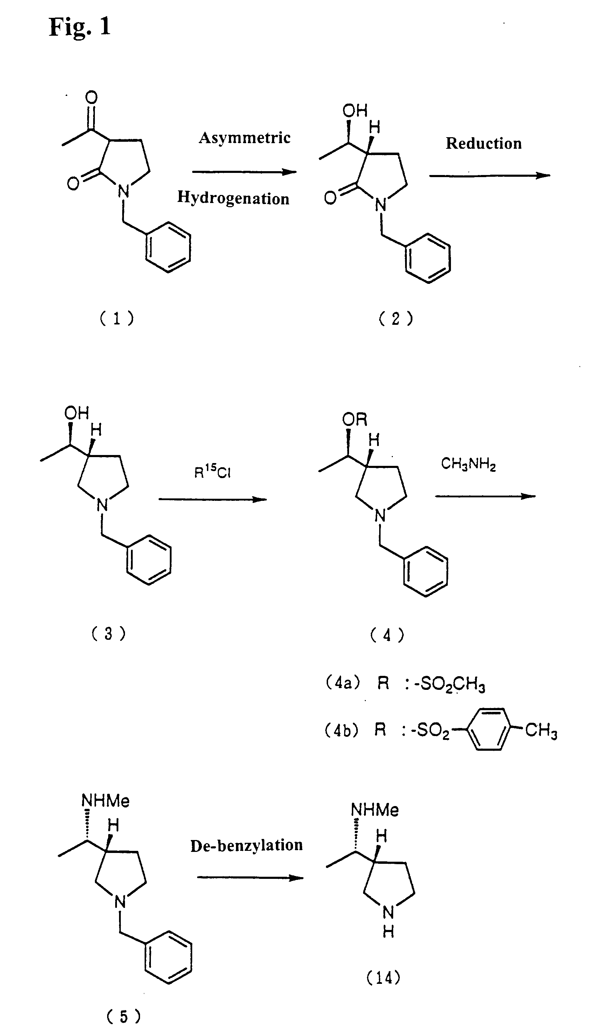

A preferable process of the present invention in which (3R,1'S)-1-benzyl-3-[(1'-N-methylamino)ethyl]pyrrolidine (5) is obtained from 3-acetyl-1-benzyl-2-pyrrolidinone (1) as the starting material is shown in FIG. 1.

The starting material in the present invention, i.e. 3-acetyl-1-benzyl-2-pyrrolidinone (1) represented by formula (1), can be obtained in a known method. For example, it can be obtained by the method described in Japanese Patent Application Laid-open No. 4-95067, that is, by reacting commercially available 1-benzyl-2-pyrrolidinone with dimethylacetamide at -60 °C in the presence of lithium diisopropylamide.

One aspect of the present invention lies in producing (3S,1'R)-1-benzyl-3-[(1'-hydroxy)ethyl]-2-pyrrolidinone (2) by asymmetric hydrogenation of 3-acetyl-1-benzyl-2-pyrrolidinone represented by formula (1) by use of a complex formed from bidentate phosphine and ruthenium as a catalyst.

The complex formed from ruthenium an optically active phosphine used as the catalyst includes:
(i) A complex represented by the following general formula (9):

   Ru ₓH_{y}Cl _{z} (L) ₂ (S) ₚ (9)

   wherein L is bidentate phosphine represented by the general formula (6): 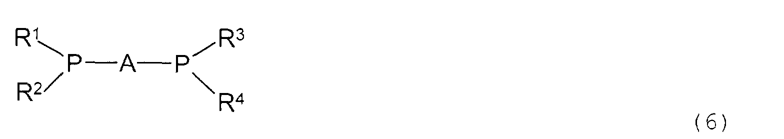 wherein R¹, R², R³, R⁴ and A have the same meanings as defined above, and S is represented by the following general formula (10):

   NR¹¹ R¹² R¹³ (10)

   wherein R¹¹, R¹² and R¹³ may be the same or different and each represent a C₁ to C₄ lower alkyl group, and when y is 0, x is 2, z is 4, and p is 1; when y is 1, x is 1, z is 1, and p is 0,
(ii) A complex represented by the following general formula (11) :

   Ru (OCOR¹⁴) ₂ (L) (11)

   wherein R¹⁴ represents a C₁ to C₄ lower alkyl group and trifluoromethyl group, and L represents the same bindentate phosphine as defined above,
(iii) A complex represented by the following general formula (12) :

   Ru X₂ (L) (12)

   wherein X represents a halogen atom, and L represents the same bidentate phosphine as defined above, and
(iv) A complex represented by the following general formula (13) :

   RuXₖJₘ (L) Qₙ (13)

   wherein X represents a halogen atom, and J represents benzene, p-cymene, C₁ to C₄ lower alkyl benzoate or acetonitrile, Q represents a halogen atom, ClO₄, PF₆ and BF₄, L represents the same bidentate phosphine as defined above; in case J is benzene, p-cymene or lower alkyl benzoate, m is 1 and n is 1 when k is 1; and in case J is acetonitrile, m is 1 and n is 1 when k is 1, and m is 4 and n is 2 when k is 0.

The ruthenium compound used as the starting material for such complexes includes e.g. [RuCl₂(COD)]ₙ (wherein COD is 1,5-cycloctadiene, n is an integer of 1 to about 1000), [RuBr₂(COD)]ₙ, [RuCl₂(NBD)]ₙ (wherein NBD is norbornadiene), [RuBr₂(NBD)]ₙ, [RuCl₂(benzene)]₂, [RuBr₂(benzene)]₂, [RuI₂(benzene)]₂, [RuCl₂(p-cymene)]₂, [RuBr₂(p-cymene)]₂, [RuI₂(p-cymene)] ₂ etc.

The optically active bindentate phosphine as another starting material for the above complexes includes e.g. the following compounds. A symbol for optical rotation, (+) or (-), is omitted for the following description of optically active bidentate phosphine.
2,2'-bis(diphenylphosphino)-6,6'-dimethylbiphenyl (referred to hereinafter as «biphemp»),
2,2'-bis(di(p-tolyl) )phosphino)-6,6'-dimethylbiphenyl (referred to hereinafter as «T-biphemp»),
2,2'-bis(di(p-tert-Bu-phenyl)phosphino)-6,6'-dimethylbiphenyl (referred to hereinafter as «Bu-biphemp»), 2,2'-bis(di(p-methoxyphenyl)phosphino)-6,6'-dimethylbiphenyl (referred to hereinafter as «MeO-biphemp»),
2,2'-bis(di(p-chlorophenyl)phosphino)-6,6'-dimethylbiphenyl (referred to hereinafter as «Cl-biphemp»),
2,2'-bis(diphenylphosphino)-5,5',6,6',7,7',8,8'-octahydro-1,1'-binaphthyl (referred to hereinafter as «H₈binap»),
2,2'-bis(di(p-(p-tolyl)phosphino)-5,5',6,6',7,7',8,8'-octahydro-1,1'-binaphthyl (referred to hereinafter as «T-H8binap»),
2,2'-bis(di(p-tert-Bu-phenyl)phosphino)-5,5',6,6',7,7',8,8'-octahydro-1,1'-binaphthyl (referred to hereinafter as «Bu-H₈binap»),
2,2' -bis (di (p-methoxyphenyl) phosphino) -5,5',6,6',7,7',8,8'-octahydro-1,1'- binaphthyl (referred to hereinafter as «MeOH₈binap»),
2,2'-bis(di(p-chlorophenyl)phosphino)-5,5',6,6',7,7',8,8'-octahydro-1,1'-binaphthyl (referred to hereinafter as «ClH₈binap») ,
2,2'-bis(diphenylphosphino)-1,1'-binaphthyl (referred to hereinafter as «binap»),
2,2'-bis(di(p-tolyl)phosphino)-1,1'-binaphthyl (referred to hereinafter as «T-binap»),
2,2'-bis(di(p-tert-Bu-phenyl)phosphino)-1,1'-binaphthyl (referred to hereinafter as «Bu-binap»),
2,2'-bis(di(xylyl)phosphino)-1,1'-binaphthyl (referred to hereinafter as «Xy-binap»),
2,2'-bis(di(p-methoxyphenyl)phosphino)-1,1'-binaphthyl (referred to hereinafter as «MeO-binap»),
2,2'-bis (di(chlorophenyl)phosphino)-1,1'-binaphthyl (referred to hereinafter as «Cl-binap»),
2,2'-bis (di(cyclohexyl)phosphino)-1-1'-binaphthyl (referred to hereinafter as «Cy-binap»),
2,2'-bis(di(cyclopentyl)phosphino)-1,1'-binaphthyl (referred to hereinafter as «Cp-binap»),
2,2'-bis(diphenylphosphino)-5,5'-diamino-1,1-binaphthyl (referred to hereinafter as «NH-binap»),
2,2'bis(diphenylphosphino)-5,5'-di(acetylamino)-1,1-binaphthyl (referred to hereinafter as «Ac-binap»),
2,2-bis(diphenylphosphino)-5,5'-disulpho-1,1'-binaphthyl (referred to hereinafter as «SO₂-binap») , and
disodium 2,2'-bis(diphenylphosphino)-1,1'-binaphthyl-5,5'-disulfonate (referred to hereinafter as «SO₃Na-binap»).

The complex used in the present invention is formed from an optically active bidentate phosphine and a ruthenium compound, and specific examples include the following compounds. A symbol for optical rotation, (+) or (-), is omitted for the following description of optically active bidentate phosphine.

The RuₓH_{y}Cl_{z}(L)₂(S)ₚ type complex includes RuHCl(biphemp)₂, RuHCl(T-biphemp)₂, RuHCl(Bu-biphemp)₂, RuHCl(MeO-biphemp)₂, RuHCl(Cl-biphemp)₂, RuHCl (H₈binap)₂, RuHCl(T-H₈binap)₂, RuHCl(Bu-H₈binap)₂, RuHCl (MeO-H₈binap)₂, RuHCl(Cl-H₈binap)₂, RuHCl(binap)₂, RuHCl(T-binap)₂, RuHCl(Bubinap)₂, RuHCl(MeO-binap)₂, RuHCl(Cl-binap)₂, RuHCl(Xybinap)₂, RuHCl(Cy-binap)₂, RuHCl(Cp-binap)₂, RuHCl(NH-binap)₂, RuHCl(Ac-binap)₂, RuHCl(SO₂-binap)₂, RuHCl(SO₃Na-binap)₂ etc., Ru₂Cl₄(biphemp)₂(NEt₃), Ru₂Cl₄(T-biphemp)₂(NEt₃), Ru₂Cl₄(Bu-biphemp)₂(NEt₃) , Ru₂Cl₄(MeO-biphemp)₂(NEt₃), Ru₂Cl₄(Cl-biphemp)₂(NEt₃), Ru₂Cl₄(H₈binap)₂(NEt₃), Ru₂Cl₄(T-H₈binap)₂(NEt₃), Ru₂Cl₄(Bu-H₈binap)₂(NEt₃), Ru₂Cl₄(MeO-H₈binap)₂(NEt₃), Ru₂Cl₄(Cl-H₈binap)₂(NEt₃), Ru₂Cl₄(binap)₂(NEt₃), Ru₂Cl₄(T-binap)₂(NEt₃), Ru₂Cl₄(Bu-binap)₂(NEt₃), Ru₂Cl₄(MeO-binap)₂(NEt₃), Ru₂Cl₄(Cl-binap)2(NEt3), Ru₂Cl₄(Xy-binap)₂(NEt₃), Ru₂Cl₄(Cy-binap)₂(NEt₃), Ru₂Cl₄(Cp-binap)₂(NEt₃), Ru₂Cl₄(NH-binap)₂(NEt₃), Ru₂Cl₄(Ac-binap)₂(NEt₃), Ru₂Cl₄(SO₂-binap)₂(NEt₃), Ru₂Cl₄(SO₃Na-binap)₂(NEt₃) etc.

The Ru(OCOR¹⁴)₂(L) type complex includes Ru(OCOCH₃)₂(biphemp), Ru (OCOCH₃)₂(T-biphemp), Ru(OCOCH₃)₂(Bu-biphemp), Ru(OCOCH₃)₂(MeO-biphemp), Ru(OCOCH₃)₂(Cl-biphemp), Ru (OCOCH₃)₂(H₈binap), Ru (OCOCH₃)₂(T-H₈binap), Ru(OCOCH₃)₂(Bu-H₈binap), Ru (OCOCH₃)₂(MeO-H₈binap), Ru (OCOCH₃)₂(Cl-H₈binap), Ru(OCOCH₃)₂(binap), Ru(OCOCH₃)₂(T-binap), Ru(OCOCH₃)₂(Bu-binap), Ru(OCOCH₃)₂(MeO-binap), Ru(OCOCH₃)₂(Cl-binap), Ru(OCOCH₃)₂(Xy-binap), Ru(OCOCH₃)₂(Cy-binap), Ru(OCOCH₃)₂(Cp-binap), Ru(OCOCH₃)₂(NH-binap), Ru(OCOCH₃)₂(Ac-binap), Ru(OCOCH₃)₂(SO₂-binap), Ru (OCOCH₃)₂(SO₃Na-binap), Ru(OCOCF₃)₂(biphemp), Ru(OCOCF₃)₂(T-biphemp), Ru(OCOCF₃)₂(Bu-biphemp), Ru(OCOCF₃)₂(MeO-biphemp), Ru(OCOCF₃)₂(Cl-biphemp), Ru (OCOCF₃)₂(H₈binap), Ru (OCOCF₃)₂(T-H₈binap), Ru(OCOCF₃)₂(Bu-H₈binap), Ru (OCOCF₃)₂(MeO-H₈binap), Ru(OCOCF₃)₂(Cl-H₈binap), Ru(OCOCF₃)₂(binap), Ru(OCOCF₃)₂(T-binap), Ru(OCOCF₃)₂(Bu-binap), Ru (OCOCF₃)₂(MeO-binap), Ru(OCOCF₃)₂(Cl-binap), Ru(OCOCF₃)₂(Xy-binap), Ru (OCOCF₃)₂(Cy-binap), Ru(OCOCF₃)₂(Cp-binap), Ru(OCOCF₃)₂(NH-binap), Ru(OCOCF₃)₂(Ac-binap), Ru(OCOCF₃)₂(SO₂-binap), Ru(OCOCF₃)₂(SO₃Na-binap), Ru (OCOt-Bu)₂(biphemp), Ru(OCOt-Bu)₂(T-biphemp), Ru(OCOt-Bu)₂(Bu-biphemp), Ru(OCOt-Bu)₂(MeO-biphemp), Ru(OCOt-Bu)₂(Cl-biphemp), Ru(OCOt-Bu)₂(H₈binap), Ru(OCOt-Bu)₂(T-H₈binap), Ru(OCOt-Bu)₂(Bu-H₈binap), Ru(OCOt-Bu)₂(MeO-H₈binap), Ru(OCOt-Bu)₂(Cl-H₈binap), Ru(OCOt-Bu)₂(binap), Ru(OCOt-Bu)₂(T-binap), Ru (OCOt-Bu)₂(Bu-binap), Ru(OCOt-Bu)₂(MeO-binap), Ru(OCOt-Bu)₂(Cl-binap), Ru(OCOt-Bu)₂(Xy-binap), Ru(OCOt-Bu)₂(Cy-binap), Ru(OCOt-Bu)₂(Cp-binap), Ru(OCOt-Bu)₂(NH-binap), Ru(OCOt-Bu)₂(Ac-binap), Ru(OCOt-Bu)₂(SO₂-binap), Ru(OCOt-Bu)₂(SO₃Na-binap) etc.

The RuX₂(L) type complex includes RuCl₂(biphemp), RuCl₂(T-biphemp), RuCl₂(Bu-biphemp), RuCl₂(MeO-biphemp), RuCl₂(Cl-biphemp), RuCl₂(H₈binap), RuCl₂(T-H₈binap), RuCl₂(Bu-H₈binap), RuCl₂(MeO-H₈binap), RuCl₂(Cl-H₈binap), RuCl₂(binap), RuCl₂(T-binap), RuCl₂(Bu-binap), RuCl₂(MeO-binap), RuCl₂(Cl-binap), RuCl₂(Xy-binap), RuCl₂(Cy-binap), RuCl₂(Cp-binap), RuCl₂(NH-binap), RuCl₂(Ac-binap), RuCl₂(SO₂-binap), RuCl₂(SO₃Na-binap), RuBr₂(biphemp), RuBr₂(T-biphemp), RuBr₂(Bu-biphemp), RuBr₂(MeO-biphemp), RuBr₂(Cl-biphemp), RuBr₂ (H₈binap), RuBr₂ (T-H₈binap), RuBr₂ (Bu-H₈binap), RuBr₂(MeO-H₈binap), RuBr₂(Cl-H₈binap), RuBr₂(binap), RuBr₂(T-binap), RuBr₂(Bu-binap), RuBr₂(MeO-binap), RuBr₂(Cl-binap), RuBr₂(Xy-binap), RuBr₂(Cy-binap), RuBr₂(Cp-binap), RuBr₂(NH-binap), RuBr₂(Ac-binap), RuBr₂(SO₂-binap), RuBr₂(SO₃Na-binap) etc.

RuXₖJₘ(L)Qₙ type complex includes [RuCl(benzene) (biphemp)]Cl, [RuI(p-cymene) (T-biphemp)]I, [RuBr(benzene)(Bu-biphemp)]Br, [RuCl(Bz-Me)(MeO-biphemp)]Cl (hereinafter, Bz-Me refers to methyl benzoate), [RuCl(benzene) (Cl-biphemp)]Cl, [RuI(benzene) (H₈binap)]I, [RuCl(benzene) (T-H₈binap)]Cl, [RuCl(p-cymene) (Bu-H₈binap)]Cl, [RuBr(benzene) (MeO-H₈binap)]Br, [RuCl(benzene) (Cl-H₈binap)]Cl, [RuI(p-cymene) (binap)]I, [RuI(p-cymene) (T-binap)]I, [RuCl(benzene) (T-binap)]Cl, [RuI(Me-Ar)(Bu-binap)]I, [RuBr(benzene)(MeO-binap)]Br, [RuCl(p-cymene) (Cl-binap)]Cl, [RuI(benzene) (T-binap)]I, [RuI(p-cymene)(Xy-binap)]I, [RuCl(benzene)(Cy-binap)]Cl, [RuI(Me-Ar)(Cp-binap)]I, [RuCl(benzene) (NH-binap)]Cl, [RuCl(benzene)(Ac-binap)]Cl, [RuBr(benzene) (SO₂-binap)]Br, [RuCl(p-cymene) (SO₃Na-binap)]Cl, [RuCl(benzene) (biphemp)]ClO₄, [RuCl(p-cymene) (T-biphemp)]ClO₄, [RuCl(benzene) (Bu-biphemp)]ClO₄, [RuCl(Me-Ar) (MeO-biphemp)]ClO₄, [RuCl(benzene) (Cl-biphemp)]ClO₄, [RuCl(benzene) (H₈binap)]ClO₄, [RuCl(benzene) (T-H₈binap)]ClO₄, [RuCl(p-cymene) (Bu-H₈binap)]ClO₄, [RuCl(benzene) (MeO-H₈binap)]ClO₄, [RuCl(benzene) (Cl-H₈binap)]ClO₄, [RuCl(p-cymene) (binap)]ClO₄, [RuCl(benzene) (T-binap)]ClO₄, [RuCl(Me-Ar) (Bu-binap)]ClO₄, [RuCl(benzene) (MeO-binap)]ClO₄, [RuCl(p-cymene) (Cl-binap)]ClO₄, [RuI(benzene) (Xy-binap)]ClO₄, [RuCl(p-cymene) (Xy-binap)]ClO₄, [RuI(benzene) (Cy-binap)]ClO₄, [RuCl(p-cymene) (Cy-binap)]ClO₄, [RuCl(Me-Ar) (Cp-binap)]ClO₄, [RuCl(benzene) (NH-binap)]ClO₄, [RuCl(benzene) (Ac-binap)]ClO₄, [RuCl(benzene) (SO₂-binap)]ClO₄, [RuCl(p-cymene) (SO₃Na-binap)]ClO₄ etc., [RuCl(CH₃CN)₂(biphemp)]Cl, [RuCl(CH₃CN)₂(T-biphemp)]Cl, [RuCl(CH₃CN)₂(Bu-biphemp)]Cl, [RuCl(CH₃CN)₂(MeO-biphemp)]Cl, [RuCl(CH₃CN)₂(Cl-biphemp)]Cl, [RuCl(CH₃CN)₂(H₈binap)]Cl, [RuCl(CH₃CN)₂(binap)]Cl, [RuCl(CH₃CN)₂(T-binap)]Cl, [RuCl(CH₃CN)₂(Cy-binap)]Cl, [Ru(CH₃CN)₄(binap) ](ClO₄)₂, [Ru(CH₃CN)₄(T-binap) (BF₄)₂, [Ru(CH₃CN)₄(binap)](PF₆)₂ etc.

These complexes can be produced using e.g. the following methods:

### (i) RuₓH_{y}Cl_{z}(L)₂(S)ₚ type complex

Synthesis of the RuₓH_{y}Cl_{z}(L)₂(S)ₚ type complex can be carried out according to the method described by T. Ikariya et al.: Journal Chemical Society Chemical Communication 922, 1985 or Japanese Patent Application Laid-open No. 61-63690. For example, in the case of the complex where y = 0, one molar equivalent of [RuCl₂(COD)]ₙ (n is an integer) obtained by reacting ruthenium chloride with 1,5-cyclooctadiene (COD) in an alcohol solvent such as ethanol etc. is reacted with 1.05 to 1.2 molar equivalents of bidentate phosphine in the presence of 4 molar equivalents of a tertiary amine in a solvent such as toluene, ethanol or the like whereby the object compound can be obtained. The complex where y =1 can be obtained by the same operation as above where bidentate phosphine L is used in a 2-fold molar excess amount relative to [RuCl₂(COD)]ₙ.

### (ii) Ru(OCOR¹⁴)₂(L) type complex

The Ru(OCOR¹⁴)₂(L) type complex can be obtained according to the method described in Japanese Patent Application Laid-open No. 61-63690. For example, Ru₂Cl₄(L)₂(NEt₃) obtained in item (i) above is reacted as a starting material with a lower carboxylate represented by R¹⁴COOM (R¹⁴ and M have the same meanings as defined above) in an alcohol solvent such as methanol, ethanol, tert-butanol etc. at a temperature of about 20 to 110 °C for 3 to 15 hours, then the solvent is distilled off, and the object complex is extracted with a solvent such as ether, ethanol etc. and then dried whereby the complex can be obtained in a crude form. A purified preparation can be obtained by further recrystallizing the crude product from ethyl acetate etc.

### (iii) RuX₂(L) type complex

This can be obtained according to the method described in Japanese Patent Application Laid-open No. 63-145292. For example, Ru₂Cl₄(L)₂(NEt₃) obtained in item (i) above is reacted with a salt represented by MQ (M and Q have the same meanings as defined above) in a mixed solvent of water and methylene chloride, using as phase-transfer catalyst a quaternary ammonium salt or quaternary phosphonium salt represented by the following general formula (15).

R¹⁶ R ¹⁷ R ¹⁸ R ¹⁹ Z X (15)

wherein R ¹⁶, R ¹⁷, R ¹⁸, and R ¹⁹ represent a C₁ to C₁₆ alkyl group, phenyl group and benzyl group, Z represents a nitrogen atom or phosphorus atom, and X represents the same meaning as defined above).

The salt used herein includes perchlorates with sodium, potassium, lithium etc., tetrafluoroborate and hexafluorophosphite, and the phase-transfer catalyst used include quaternary ammonium salts such as tetramethyl ammonium bromide, tetrapropyl ammonium bromide, tetrabutyl ammonium chloride, tetrabutyl ammonium iodide, octyl trimethyl ammonium bromide, lauryl trimethyl ammonium bromide, lauryl triphenyl ammonium bromide, cetyl trimethyl ammonium bromide, methyl trioctyl ammonium chloride and benzyl triethyl ammonium bromide, as well as their corresponding quaternary phosphonium salts.

### (iv) RuXₖ Jₘ(L)Qₙ type complex

This can be obtained according to the method described in Japanese Patent Application Laid-open No. 2-192189. For example, the complex where J is benzene, p-cymene or lower alkyl benzoate, can be obtained in the following manner. The above complex in which both X and Q are chlorine atoms can be obtained by reacting [RuCI₂J]₂ as a starting material with bidentate phosphine L at 25 to 50°C for 30 minutes to 3 hours in a single solvent such as methanol, ethanol, benzene, methylene chloride etc. or in a mixed solvent thereof, and then distilling off the solvent under reduced pressure. In the case where both X and Q are bromine atoms or iodine atoms, [RuCI₂J]₂ is reacted as a starting material with a salt represented by MBr or MI (M has the same meaning as defined above), using water as solvent or using a phase-transfer catalyst represented by the general formula (14) in a mixed solvent of water and methylene chloride, whereby [RuBr₂J]₂ or [RuI₂J]₂ is obtained. Then, the resulting [RuBr₂J]₂ or [RuI₂J]₂ is reacted at 25 to 50 °C for 30 minutes to 3 hours with bidentate phosphine L in a single solvent such as methanol, ethanol, benzene, methylene chloride etc. or in a mixed solvent thereof, and the solvent is then distilled off under reduced pressure whereby the object complex can be obtained. In the case where Q is other than halogen atom, a complex such as [RuCl(J) (L)]Cl obtained above is dissolved in methanol, ethanol, benzene, methylene chloride etc., and a salt represented by MQ' (M has the same meaning as defined above, and Q' represents ClO₄, BF₄ and PF₆) is added to it, then the mixture is stirred, a small amount of insolubles are filtered off, and the filtrate is concentrated and dried whereby the object complex can be obtained. In the case of the complex where J is acetonitrile, a complex such as [RuCl(J) (L)]Cl is dissolved in acetonitrile and refluxed at 50 °C for 10 to 24 hours, and excess acetonitrile is distilled off, and the resulting product is dried. This crude complex is recrystallized from methylene chloride whereby [RuCl(CH₃CN) (L)]Cl can be obtained. For example, [RuCl(J)(L)]Cl complex is dissolved in a mixed solvent of acetonitrile and methanol, ethanol, benzene, methylene chloride or the like, and MQ' (M has the same meaning as defined above) is added to it, then the mixture is stirred at 25 to 50 °C for 10 to 24 hours, the solvent is distilled off, and the product is dried and then recrystallized from methylene chloride, whereby a complex such as [Ru(CH₃CN)₄(L)](ClO₄)₂ can be obtained.

To carry out the asymmetric hydrogenation of the present invention, 3-acetyl-1-benzyl-2-pyrrolidinone (Compound (1)) is dissolved in a single solvent selected from alcohols such as methanol, ethanol, isopropanol etc., ketones such as acetone, methyl ethyl ketone, methyl isobutyl ketone, cyclohexanone etc., ethers such as tetrahydrofuran, 1,3-dioxolane, diethyl ether, di-isopropyl ether etc., esters such as ethyl acetate, butyl acetate etc., aromatic hydrocarbons such as benzene, toluene, xylene etc., halogenated hydrocarbons such as methylene chloride etc., or a mixture thereof and subjected to asymmetric hydrogenation in an autoclave. In this reaction, a ruthenium/bidentate phosphine complex is added in an amount of 1/10000 to 1/10 mole, preferably 1/2000 to 1/100 mole, per mole of Compound (1) as the substrate, and hydrogenation is carried out with stirring at a hydrogen pressure of 5 to 100 kg/cm², preferably 30 to 60 kg/cm² , at a reaction temperature of 25 to 100°C, preferably 40 to 70°C. The time required for this reaction is 3 to 100 hours, preferably 8 to 24 hours, depending on the reaction temperature. The resulting optically active 1-benzyl-3-[(1'-hydroxy)ethyl]-2-pyrrolidinone can be purified to high degrees in terms of optical purity and diastereomeric purity by recrystallization from an organic solvent such as toluene, ethyl acetate, acetone or the like.

Then, (3S,1'R)-1-benzyl-3-[(1'-hydroxy)ethyl]-2-pyrrolidinone (Compound (2) obtained in the manner described above is reduced with a usual amide group-reducing reagent to be converted into the corresponding amine i.e.(3R,1'R)-1-benzyl-3-[(1'-hydroxy)ethyl]-2-pyrrolidine. Specifically, it is reduced with a reducing agent such as lithium aluminum hydride, borane etc. or by a combination of sodium borohydride and Lewis acid, as described by M. Hudliky in Reduction in Organic Chemistry (John Wiley & Sons Ltd.). Further, reduction of a side-chain amide by sodium borohydride and sulfuric acid is known as described in Japanese Patent Application Laid-open No. 8-169877, but in the present invention, it was found that pyrrolidinone i.e. a cyclic amide group can be reduced into pyrrolidine. As the solvent for this reaction, use is made of ethers such as diethyl ether, di-isopropyl ether, dimethoxy-ethane, tetrahydrofuran etc. among which tetrahydrofuran is preferably used. In the case of lithium aluminum hydride, the amount of the reducing agent is 1 to 4 molar equivalents, preferably 2 molar equivalents, relative to Compound (2) as the substrate. The reaction temperature ranges from 0 to 70 °C depending on the solvent used. If sodium borohydride and sulfuric acid are used in the present invention, sodium borohydride is used in an amount of 2 to 4 molar equivalents, preferably 3 molar equivalents, and sulfuric acid is used in an amount 1 to 2 molar equivalents, preferably 1.5 molar equivalents, relative to Compound (2) as the substrate. The reaction temperature ranges from 0 to 50 °C depending on the solvent used. If sodium borohydride and sulfuric acid are used, 1 molecule of borane is bound to the reduced product (amine compound) to form a borane complex, and this product is heating-treated with a mineral acid such as hydrochloric acid or sulfuric acid to give a free amine compound (3R,1'R)-1-benzyl-3-[(1'-hydroxy)ethyl]-2-pyrrolidine. Although this product is used in the subsequent step without purification, a highly purified preparation can be obtained through vacuum distillation.

Then, (3R,1'R)-1-benzyl-3-[(1'-hydroxy)ethyl] ] pyrrolidine (Compound (3)) obtained in the manner described above is reacted with methanesulfonyl chloride or p-toluene sulfonyl chloride, preferably in the presence of a tertiary amine, whereby Compound (4) i.e. (3R,1'R)-1-benzyl-3-[(1'-methanesulfonyloxy)ethyl]pyrrolidine or (3R,1'R)-1-benzyl-3-[(1'-p-toluenesulfonyloxy)ethyl]pyrrolidine can be obtained. As the tertiary amine, use are made of triethyl amine, tributyl amine, ethyl di-isopropyl amine, dimethyl aniline, pyridine, N-methylpiperidine etc., among which triethyl amine is particularly preferable. The amount of the tertiary amine is at least equivalent molar relative to Compound (3) as the substrate. The reaction temperature is in the range of 0 to 35 °C, preferably 10 to 20 °C.

Then, the resulting product (3R,1'R)-1-benzyl-3-[(1'-methanesulfonyloxy)ethyl]pyrrolidine or (3R,1'R)-1-benzyl-3-[(1'-p- toluenesulfonyloxy)ethyl]pyrrolidine is used in the subsequent step without purification. That is, (3R,1'R)-1-benzyl-3-[(1'-methanesulfonyloxy)ethyl] pyrrolidine (4a) or (3R,1'R)-1-benzyl-3-[(1'-p-toluenesulfonyloxy)ethyl] pyrrolidine (4b), or both of the compounds, are reacted under heating with methylamine in an organic solvent whereby (3R,1'S)-1-benzyl-3-[(1'-N-methylamino)ethyl]pyrrolidine can be obtained as Compound (5). As the solvent, use is made of aliphatic hydrocarbons such as hexane, heptane, cyclohexane etc., aromatic hydrodarbons such as benzene, toluene, xylene etc., alcohols such as methanol, ethanol, isopropanol, tert-butanol etc., amides such as dimethylformamide, dimethylacetamide etc., nitriles such as acetonitrile etc. or ethers such as diethyl ether, di-isopropyl ether, dimethoxy ethane, tetrahydrofuran etc., and these can be used singly or in combination. Tetrahydrofuran is preferably used. The reaction temperature is in the range of 10 to 120 °C, preferably 60 to 90°C. The reaction time is in the range of 4 to 48 hours, preferably 8 to 40 hours. The amount of methylamine is 10 to 50 % by weight, preferably 30 % by weight, relative to the solvent. In this methyl-amination, inversion at the 1'-position occurs in its configuration. Although olefin compounds from which a mesyl group or tosyl group has been eliminated occur as by-products, (3R,1'S)-1-benzyl-3-[(1'-N-methy-lamino)-ethyl]pyrrolidine (5) can be obtained with high purity through purification using vacuum distillation.

(3R,1'S)-1-benzyl-3-[(1'-N-methylamino)ethyl]-pyrrolidine (5) is further subjected de-benzylation by a general method for removal of a protective group, described in Protective Group in Organic Synthesis (published by John Wiley & Sons, 1981), for example by hydrogenation using a palladium carbon etc. as a catalyst so that (3R,1'S)-3-[(1'-N-methylamino)ethyl]pyrrolidine (14) can be obtained.

According to the present invention, (3R,1'S)-3-[(1'-Nmethylamino)ethyl]pyrrolidine that is an intermediate for synthesis of quinolone type anti-fungus agents can be obtained with higher purity and in higher yield by a safe process with fewer steps than in conventional processes.

### EXAMPLES

Hereinafter, the present invention is described in more detail by reference to the Examples, which however are not intended to limit the present invention. The physical data in the Examples were obtained using the following measurement instruments and conditions:
A) Chemical purity, optical purity and diastereomeric selectivity:
   Gas chromatography HP-5890 (Hewlett Packard Ltd.)
   Column: Silicon NB-1 (0.25 mm 30 m) produced by G. L. Science
   Temperature: a gradient of from 100 to 220°C at a rate of 10°C /min.
   Liquid chromatography L-6000 pump, L-4000 UV (Hitachi, Ltd.)
   Column-1: Inertosil ODS-2 (4.6 mm 250 mm)
   Solvent: acetonitrile/water = 7/3, pH 2.3 (phosphate)
   Flow rate: 0.5 ml/min.
   Detection 254 nm
   Column-2: CHIRALPAK AD (4.6 mm 250 mm)
   Solvent: hexane/isopropanol/ethanol = 90/5/5
   Flow rate: 0.5 ml/min.
   Detection: 220 nm
B) Proton nuclear magnetic resonance spectrum (¹H-NMR) : AM-400 (Bruker Co., Ltd.), internal standard; tetramethyl silane
C) Specific rotation: DIP-4 (Nihon Bunkoh Co., Ltd)
D) Melting point: MP-S3 (Yanagimoto Shoji K.K.)

### Example 1

### Synthesis of (3S,1'R)-1-benzyl-3-[(1'-hydroxy)ethyl]-2-pyrrolidinone (2)

A 1000-ml autoclave was charged with 113.1 g (521 mM) 3-acetyl-1-benzyl-2-pyrrolidinone (1) , 340 ml methylene chloride and 0.76 g (0.65 mM) [RuI(p-cymene) {(+) -T-binap}] I, and the mixture was reacted at a hydrogen pressure of 50 kg/cm² at 50 to 55°C for 21 hours. The optical yield and diastereomeric selectivity by HPLC column-2 were 87.9%ee and 98.8 %de, respectively. The solvent was recovered under reduced pressure, and the residue was dissolved with 340 ml toluene, stirred, dissolved, and crystallized at 0°C. The precipitated crystal was filtered to give 95.6 g of the title compound (2) as white crystal (yield, 83.7 %; chemical purity by gas chromatography, 99 %; optical purity by HPLC column-2, 99 %ee; diastereomeric selectivity by HPLC column-2, 99 %de; and melting point, 82 to 82.5°C).

Specific rotation [α ]_{D} ²³ = -10.43(c = 1.15, CHCI₃)

¹H-NMR (CDCl₃, ppm) 1.18 (3H, d, J = 6.6 Hz) , 1.91-2.04 (2H, m), 2.52-2.62 (1H, m), 3.36 (1H, d, J = 5.6 Hz), 3.12-3.20 (2H, m), 4.21-4.38 (1H, m), 4.42 (2H, s), 7.16-7.29 (5H, m).

### Examples 2 to 9

Synthesis was carried out in the same manner as in Example 1 except that isopropanol, acetone, tetrahydrofuran, methyl ethyl ketone, methyl isobutyl ketone, cyclohexanone, ethyl acetate and butyl acetate were used respectively as the solvent in place of methylene chloride. The results are shown in Table 1.

**Table 1**

| | Solvent | Diastereomeric Selectivity | Optical Purity |
|---|---|---|---|
| | | | |
| Example 2 | isopropanol | 95.5 %de | 47.5 %ee |
| Example 3 | acetone | 97.1 %de | 74.0 %ee |
| Example 4 | tetrahydrofuran | 96.1 %de | 81.5 %ee |
| Example 5 | methyl ethyl ketone | 95.7 %de | 82.4 %ee |
| Example 6 | methyl isobutyl ketone | 93.6 %de | 81.5 %ee |
| Example 7 | cyclohexanone | 93.9 %de | 80.7 %ee |
| Example 8 | ethyl acetate | 94.2 %de | 83.8 %ee |
| Example 9 | butyl acetate | 95.5 %de | 81.0 %ee |

### Examples 10 to 13

Synthesis was carried out in the same manner as in Example 1 except that [RuI(p-cymene)((+)-binap}]I, Ru₂Cl₄[(+)-binap]₂NEt₃, Ru(OCOCH₃)₂[(+)-binap] and [RuI(p-cymene)]{(+)-Xy-binap}I were used respectively as the asymmetric hydrogenation catalyst in place of [RuI(p-cymene) {(+)-T-binap}]I, and that the reaction time was varied. The results are shown in Table 2.

**Table 2**

| | Catalyst | Reaction Time | Diastereomeric | Optical Selectivity |
|---|---|---|---|---|
| Example 10 | [Rul(p-cymene) {(+) -binap } ]I | 17 h | 99.2 %de | 89.3 %ee |
| Example 11 | Ru₂Cl₄ [ (+) - binap]₂ NEt₃ | 89 h | 99.0 %de | 82.5 %ee |
| Example 12 | Ru(OCOCH₃)₂[ (+) -binap] | 51 h | 52.5 %de | 54.3 %ee |
| Example 13 | [Rul(p-cymene)] | 41 h | 93.0 %de | 86.1 %ee |
| | {(+)-Xy-binap}]I | | | |

Similarly, when Ru₂Cl₄[(+)-T-binap]₂NEt₃ was used as the asymmetric hydrogenation catalyst, the reaction time was 80 hours, the diastereomeric selectivity was 98.0 %de and the optical purity was 80.0 %ee, and when Ru₂Cl₄[(+)-biphemp]₂NEt₃ was used, the reaction time was 65 hours, the diastereomeric selectivity was 95.5 %de and the optical purity was 78.5 %ee, and when [RuI(p-cymene) { (+) -H₈binap} ] I was used, the reaction time was 35 hours, the diastereomeric selectivity was 93.5 %de and the optical purity was 82.0%ee.

### Example 14

### Synthesis of (3R,1'R)-1-benzyl-3-[(1'-hydroxy)ethyl]-pyrrolidine (3)

85.9g (0.393 M) of (3S,1'R)-1-benzyl-3-[(1'-hydroxy)ethyl]-2- pyrrolidinone (2) obtained in Example 1 were dissolved in 430 ml tetrahydrofuran and cooled at 10 to 15°C, and 44.5 g (1.2 M) of sodium borohydride were added all at once. Further, 57.7 g (0.589 M) of conc. sulfuric acid were added dropwise to it over a period of 65 minutes. Thereafter, it was heated at 40 to 60°C for 4 hours. The reaction solution was cooled and the tetrahydrofuran was removed under reduced pressure. Then, 200 ml ice-cold water were added carefully, and 400 ml of 6 N hydrochloric acid and 200 ml water were added, and the mixture was stirred under heating at 50°C for 2 hours. After cooling, the solution was adjusted to pH 10-12 with sodium hydroxide, and then extracted with 900 ml ethyl acetate. The extract was washed with 500 ml water and then dried over magnesium sulfate anhydride, and the solvent was recovered under reduced pressure, whereby 72.0 g of the title compound (3) were obtained as an oily residue [chemical purity by HPLC-1 column, 99 %; yield, 89 %; boiling point, 107 to 110°C /0.3 mmHg).

Specific rotation [α ]_{D} ²³ = +9.00 (c = 1.1, CHCI₃)

¹H-NMR (CDCl₃, ppm) 1.08 (3H, d, J = 6.3 Hz), 1.79-1.91 (2H, m), 2.08-2.11 (1H, m), 2.21-2.28 (1H, m), 2.42-2.46 (1H, m), 2.66-2.69 (1H, m), 3.58 (1H, s) , 3.83-3.89 (1H, m), 7.24-7.32 (5H, m).

### Example 15

### Synthesis of (3R,1'R)-1-benzyl-3-[(1'-hydroxy)ethyl] pyrrolidine (3)

10 g (45.6 mM) of (3S,1'R)-1-benzyl-3-[(1'-hydroxy)ethyl]-2- pyrrolidinone (2) obtained in Example 1 were dissolved in 20 ml tetrahydrofuran, then cooled at 10 to 15°C and added dropwise to a suspension of 3.47 g (91 mM) of lithium aluminum hydride in 50 ml tetrahydrofuran over a period of 30 minutes. Thereafter, the mixture was refluxed under heating at 70°C for 2 hours. The reaction solution was cooled and the tetrahydrofuran was removed under reduced pressure. Then, 10 % aqueous sodium hydroxide solution and 60 ml water were added carefully to it. The product was extracted with 200 ml ethyl acetate, then washed with water, and dried over magnesium sulfate anhydride, and the solvent was recovered under reduced pressure, whereby 8.9 g of the title compound (3) were obtained as an oily residue (yield, 95 %).

### Example 16

### Synthesis of (3R,1'R)-1-benzyl-3-[(1'-methanesulfonyloxy)ethyl]pyrrolidine (4a)

75.0 g (0.366 M) of (3R,1'R)-1-benzyl-3-[(1'-hydroxy)ethyl] pyrrolidine (3) obtained in Example 15 were dissolved in 360 ml toluene, and 61.1 ml (44.4 g, 0.439 M) were added to it, and the mixture was cooled at 10°C or less. A mixture of 33.9 ml (50.2 g, 0.439 M) methanesulfonyl chloride and 35 ml toluene was added to it over a period of 40 minutes. It was stirred as such for 1 hour, and 400 ml water were added to it, and the organic layer was extracted. The aqueous layer was adjusted to pH 9 with sodium hydroxide, further extracted with 300 ml toluene and combined with the organic layer, and the combined extract was washed with 10 % brine. The product was dried over magnesium sulfate anhydride, and the solvent was recovered under reduced pressure whereby 98.4 g of the title compound (4a) were obtained as an oily residue (chemical purity by HPLC-1 column, 94.8 %; yield, 95%).

¹H-NMR (CDCl₃, ppm) 1.39 (3H, d, J = 6.3 Hz), 1.70-1.77 (1H, m) , 2.00-2.03 (1H, m), 2.27-2.31 (1H, m), 2.45-2.47 (1H, m), 2.51-2.57 (1H, m), 2.70-2.80 (2H, m) 2.98 (3H, s), 3.62 (1H, d, J = 12.9 Hz), 3.67 (1H, d, J = 12.9 Hz), 4.69-4.76 (1H, m), 7.24-7.34 (5H, m).

### Example 17

### Synthesis of (3R,1'R)-1-benzyl-3-[(1'-paratoluenesulfonyloxy)ethyl]-pyrrolidine (4b)

4.1 g (20 mM) of (3R, 1'R)-1-benzyl-3-[(1'-hydroxy)ethyl]pyrrolidine (3) obtained in Example 15 were dissolved in 25 ml toluene, and 2.78 ml (2.02 g, 20 M) of triethyl amine were added to it, and the mixture was cooled to 10°C or less. 3.81 g (20 mM) of p-toluenesulfonyl chloride were added to it. It was stirred as such for 18 hours, and then 80 ml water were added so that the organic layer was extracted and washed with 10 % brine. The product was dried over magnesium sulfate anhydride, and the solvent was recovered under reduced pressure, whereby 6.45 g of the title compound (4b) were obtained as an oily residue (yield: 90 %).

¹H-NMR (CDCl₃, ppm) 1.13 (3H, d,J = 6.3 Hz), 1.62-1.72 (2H, m), 1.91-2.00 (1H, m), 2.01-2.11 (1H, m), 2.44 (3H, s), 2.90-2.95 (1H, m), 3.14-3.20 (2H, m), 3.27-3.32 (2H, m), 3.55-3.62 (1H, m), 7.28-7.35 (5H, m), 7.70 (2H, d, J = 1.8 Hz), 7.72 (2H, d, J = 1.8 Hz).

### Example 18

### Synthesis of (3R,1'S)-1-benzyl-3-[(1'-N-methylamino)ethyl] pyrrolidine (5)

A previously cooled pressure-resistant vessel, 500 ml, was charged with 33.5 g methylamine, followed by adding 90 ml tetrahydrofuran solution (methylamine concentration: 30 wt%) of 50.9 g (0.18 M) of (3R,1'R)-1-benzyl-3-[(1'-methanesulfonyloxy)ethyl]pyrrolidine (4a) obtained in Example 16, and the mixture was stirred under heating at 80 °C for 15 hours. The selectivity of (3R,1'S)-1-benzyl-3-[(1'-N-methylamino)ethyl]pyrrolidine (5) by gas chromatography was 77.1 %. After cooling, the solvent was recovered under reduced pressure, and 250 ml toluene and 250 ml water were added to the remaining crystal and oily residue, and the toluene layer thus separated was concentrated under reduced pressure to give 38.2 g of an oily residue. This residue was subjected to vacuum distillation to give 31.8 g of the title compound (5) (boiling point, 115 to 120 °C /1.5 mmHg; chemical purity by gas chromatography, 85.9 %; yield, 58.5 %).

¹H-NMR (CDCl₃, ppm) 1.02 (3H, d, J = 6.2 Hz), 1.46-1.54 (1H, m), 1.87-1.95 (1H, m), 2.09-2.19 (1H, m), 2.25-2.27 (1H, m), 2.37 (3H, s), 2.37-2.46 (2H, m), 2.62-2.66 (1H, m), 2.74-2.79 (1H, m), 3.57 (1H, d, J = 12.9 Hz), 3.61 (1H, d, J = 12.9 Hz), 7.21-7.33 (5H, m).

### Example 19

### Synthesis of (3R,1'S)-1-benzyl-3-[(1'-N-methylamino)ethyl] pyrrolidine (5)

A pressure-resistant vessel, 100 ml, was charged with 12 ml methanol solution containing 40 wt% methylamine, followed by adding 10 ml tetrahydrofuran solution containing 4.0 g (11 mM) of (3R,1'R)-1-benzyl-3-[(1'-p-toluenesulfonyloxy)ethyl]pyrrolidine (4b) obtained in Example 17, and the mixture was stirred under heating at 80 °C for 18 hours. After cooling, the solvent was recovered under reduced pressure, and 50 ml toluene and 50 ml water were added to the remaining oily residue, and the toluene layer thus separated was concentrated under reduced pressure to give 2.9 g of an oily residue. This residue was subjected to vacuum distillation to give 1.2 g of the title compound (5) (boiling point, 100 to 120°C /1.5 mmHg; chemical purity by gas chromatography, 69.1%; yield, 34.5%).

### Examples 20 to 31

Synthesis was carried out in the same manner as in Example 18 except that toluene, xylene, methanol, acetonitrile, 1,3-dioxolane, di-isopropyl ether, dimethoxyethane, dibutyl ether, tertiary butyl methyl ether, dimethylformamide, monomethylformamide, and monomethylacetamide were used respectively as the solvent in place of tetrahydrofuran, and that the concentration of methylamine used was 20% by weight.
The results are shown in Table 3.

**Table 3**

| | Solvent | Selectivity of Compound (5) |
|---|---|---|
| Example 20 | toluene | 58.5 % |
| Example 21 | xylene | 49.0 % |
| Example 22 | methanol | 45.5 % |
| Example 23 | acetonitrile | 16.7 % |
| Example 24 | 1,3-dioxolane | 31.4 % |
| Example 25 | di-isopropyl ether | 19.0 % |
| Example 26 | dimethoxyethane | 66.5 % |
| Example 27 | dibutyl ether | 35.0 % |
| Example 28 | t-butylmethyl ether | 54.8 % |
| Example 29 | dimethylformamide | 40.4 % |
| Example 30 | monomethylformamide | 23.8 % |
| Example 31 | monomethylacetamide | 40.9 % |

### Example 32 to 35

Synthesis was carried out in the same manner as in Example 18 except that the concentration of methylamine was varied. The results are shown in Table 4.

**Table 4**

| | Concentration of Methylamine | Selectivity of Compound (5) |
|---|---|---|
| Example 32 | 10 wt% | 60.7 % |
| Example 33 | 20 wt% | 72.2 % |
| Example 34 | 40 wt% | 68.2 % |
| Example 35 | 50 wt% | 64.9 % |

### Reference Example 1

### Synthesis of (3R,1'S)-3-[(1'-N-methylamino)ethyl]pyrrolidine (14)

A 1000-ml autoclave was charged with 4.5 g of 5% palladium on carbon and a solution prepared by dissolving, in 225 ml methanol, 45.0 g (0.206 M; chemical purity, 85.9%) of (3R,1'S)-1-benzyl-3-[(1'-N-methylamino)ethyl]pyrrolidine (5) obtained in Example 18, and the mixture was stirred under heating at 65°C for 18 hours at a hydrogen pressure of 30 atm. The catalyst was removed by filtration and the solvent was recovered under reduced pressure, and the remaining oily residue, 37.9 g, was subjected to vacuum distillation to give 20.3 g of the title compound (14) (yield, 85 %; boiling point, 80 to 82°C/11 mmHg; chemical purity by gas chromatography, 95.3%).

Specific rotation [α]_{D} ²⁴ = +37.25 (c =1.16, EtOH)

¹H-NMR, (CDCl₃, ppm) 1.06 (3H, d, J = 6.2 Hz), 1.36-1.45 (1H, m), 1.72 (1H, br. s), 1.81-1.89 (1H, m), 1.95-2.06 (1H, m), 2.40 (3H, s), 2.38-2.45 (2H, m), 2.59-2.64 (1H, m), 2.86-2.97(2H, m) , 3.08-3.13 (1H, m).

## Claims

1. A process for producing (3R,1'S)-1-benzyl-3-[(1'-N-methylamino)ethyl]pyrrolidine represented by formula (5) : 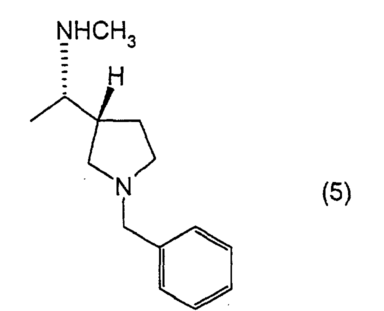 **characterised in that** said process comprises asymmetric hydrogenation of 3-acetyl-1-benzyl-2-pyrrolidinone represented by formula (1) : 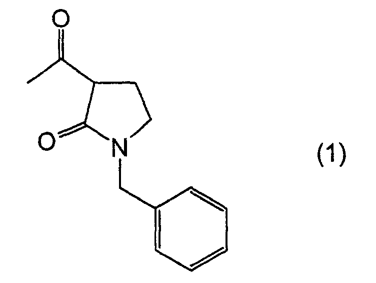 by use of a complex formed from bidentate phosphine and ruthenium as a catalyst to produce (3S,1'R)-1-benzyl-3-[(1'-hydroxyl)ethyl]-2-pyrrolidinone represented by formula (2) : 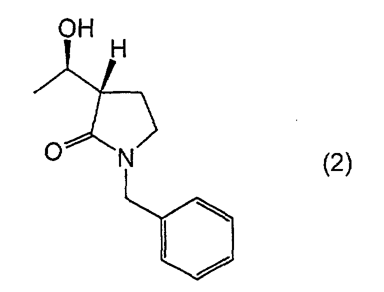 reducing the resulting (3S,1'R)-1-benzyl-3-[(1'-hydroxyl)ethyl]-2-pyrrolidinone to produce (3R,1'R)-1-benzyl-3-[(1'-hydroxy)-ethyl]pyrrolidine represented by formula (3) : 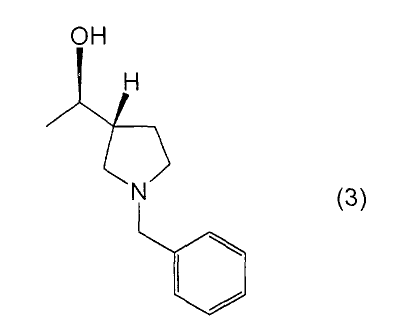 mesylating and/or tosylating the resulting (3R,1'R)-1-benzyl-3-[(1'-hydroxy)ethyl]pyrrolidine to produce (3R,1'R)-1-benzyl-3-[(1'-methanesulfonyloxy)ethyl]pyrrolidine and/or (3R,1'R)-1-benzyl-3-[(1'-p-toluenesulfonyloxy)ethyl]pyrrolidine represented by formula (4): 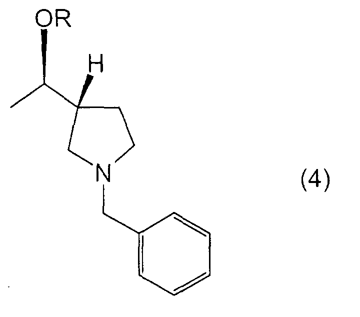 wherein R represents a methanesulfonyl group or p-toluenesulfonyl group, and methylaminating the resulting (3R,1'R)-1-benzyl-3-[(1'-methanesulfonyloxy)ethyl]pyrrolidine or (3R,1'R)-1-benzyl-3-[(1'-toluenesulfonyloxy)ethyl]pyrrolidine.

2. The process for producing (3R,1'S)-1-benzyl-3-[(1'-N-methylamino)ethyl]pyrrolidine according to claim 1, wherein the bidentate phosphine is a bidentate phosphine represented by the following general formula (6): 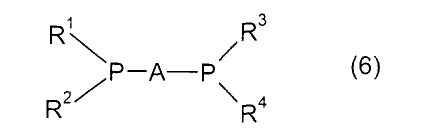 wherein R¹, R², R³ and R⁴ may be the same or different and each represent a phenyl group, cyclohexyl group, cyclopentyl group, C₁ to C₄ lower alkyl group, C₁ to C₄ lower alkoxy group, or halogen-substituted phenyl group;
A is represented by the following general formula (7): 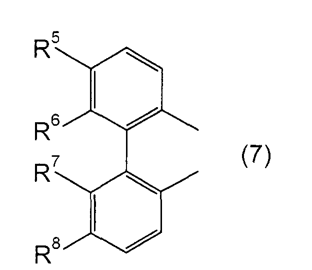 wherein R⁵ and R⁸ each represent a hydrogen atom while R⁶ and R⁷ each represent a methyl group, or R⁵ and R⁶ or R⁷ and R⁸ may be combined to form a tetramethylene group, or is represented by the following general formula (8): 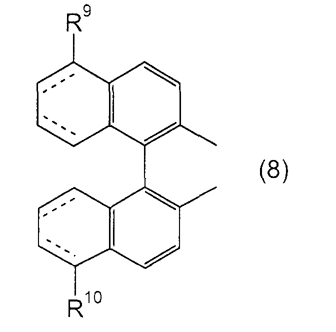 wherein R⁹ and R¹⁰ may be the same or different and each represent a hydrogen atom, amino group, acetylamino group, or -SO₃M whereupon M stands for a hydrogen atom or alkali metal atom, and the combination of a full line and a broken line signifies either a saturated bond or a double bond.

3. The process for producing (3R,1'S)-1-benzyl-3-[(1'-N-methylamino)ethyl]pyrrolidine according to claim 1 or 2, wherein the complex formed from bidentate phosphine and ruthenium is a complex represented by the following general formula (9) :
RuₓH_{y}Cl_{z}(L)₂(S)ₚ (9)
wherein L is a bidentate phosphine represented by the following general formula (6) : 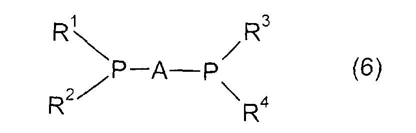 wherein R¹, R², R³, R⁴ and A have the same meanings as defined in claim 2, and S is represented by the following general formula (10) :
NR¹¹R¹²R¹³ (10)
wherein R¹¹, R¹² and R¹³ may be the same or different and each represent a lower alkyl group, and when y is 0, x is 2, z is 4, and p is 1; when y is 1, x is 1, z is 1, and p is 0.

4. The process for producing (3R,1'S)-1-benzyl-3-[(1'-N-methylamino)ethyl]pyrrolidine according to claim 1 or 2, wherein the complex formed from bidentate phosphine and ruthenium is a complex represented by the following general formula (11):
Ru(OCOR¹⁴)₂(L) (11)
wherein R¹⁴ represents a C₁ to C₄ lower alkyl group and trifluoromethyl group, and L represents the same bidentate phosphine of formula (6) in claim 2 or 3.

5. The process for producing (3R,1'S)-1-benzyl-3-[(1'-N-methylamino)ethyl]pyrrolidine according to claim 1 or 2, wherein the complex formed from bidentate phosphine and ruthenium is a complex represented by the following general formula (12):
RuX₂(L) (12)
wherein X represents a halogen atom, and L represents the same bidentate phosphine of formula (6) as defined in claim 2 or 3.

6. The process for producing (3R,1'S)-1-benzyl-3-[(1'-N-methylamino)ethyl]pyrrolidine according to claim 1 or 2, wherein the complex formed from bidentate phosphine and ruthenium is a complex represented by the following general formula (13):
RuXₖJₘ(L)Qₙ (13)
wherein X represents a halogen atom, and J represents benzene, p-cymene, C₁ to C₄ lower alkyl benzoate or acetonitrile, Q represents a halogen atom, ClO₄, PF₆ and BF₄, L represents the same bidentate phosphine of formula (6) as defined in claim 2 or 3; in case J is benzene, p-cymene or lower alkyl benzoate, m is 1 and n is 1 when k is 1; and in case J is acetonitrile, m is 1 and n is 1 when k is 1, and m is 4 and n is 2 when k is 0.

7. The process for producing (3R,1'S)-1-benzyl-3-[(1'-N-methylamino)ethyl]pyrrolidine according to any one of claims 1 to 6, wherein (3S,1'R)-1-benzyl-3-[(1'-hydroxy)ethyl]-2-pyrrolidinone represented by formula (2) : 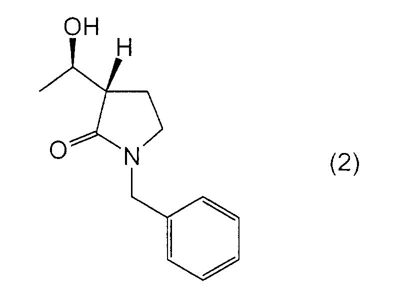 is reduced with lithium aluminium hydride, borane, sodium borohydride and a mineral acid or with sodium borohydride and an organic acid.

8. The process for producing (3R,1'S)-1-benzyl-3-[(1'-N-methylamino)ethyl]pyrrolidine according to any one of claims 1 to 7, wherein (3S,1'R)-1-benzyl-3-[(1'-hydroxy)ethyl]-2-pyrrolidine represented by formula (3) : 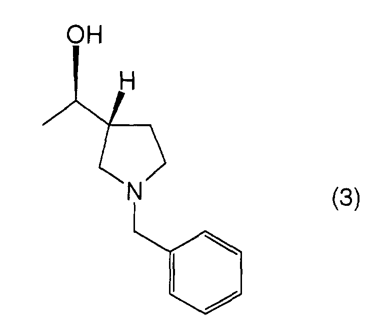 is reacted with methanesulfonyl chloride or p-toluenesulfonyl chloride in the presence of a base.

9. The process for producing (3R,1'S)-1-benzyl-3-[(1'-N-methylamino)ethyl]pyrrolidine according to any one of claims 1 to 8, wherein (3S,1'R)-1-benzyl-3-[(1'-methanesulfonyloxy)ethyl]-2-pyrrolidine and/or (3R,1'R)-1-benzyl-3-[1'-p-toluenesulfonyloxy)ethyl]pyrrolidine represented by formula (4): 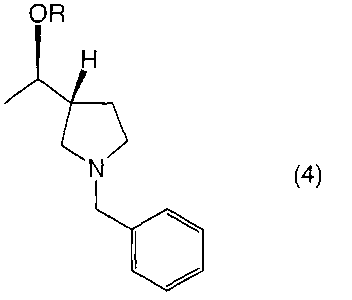 is reacted with methylamine in an organic solvent.

## Patentansprüche

1. Verfahren zur Herstellung von (3R,1'S)-1-Benzyl-3-[(1'-N-methylamino)ethyl]-pyrrolidin der Formel (5): 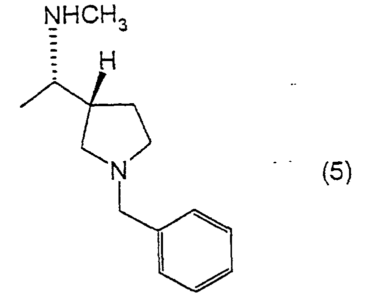 **dadurch gekennzeichnet, daß** das Verfahren asymmetrisches Hydrieren von 3-Acetyl-1-benzyl-2-pyrrolidinon der Formel (1): 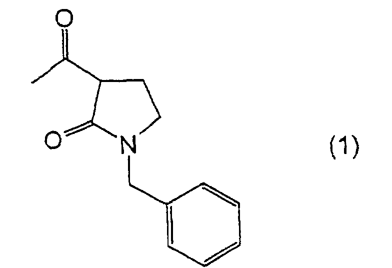 unter Verwendung eines aus Bidentatphosphin und Ruthenium gebildeten Komplexes als Katalysator, um (3S,1'R)-1-Benzyl-3-[(1'-hydroxy)ethyl]-2-pyrrolidinon der Formel (2) herzustellen: 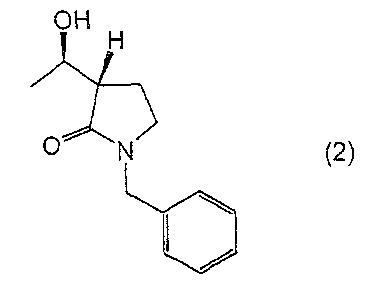 Reduzieren des resultierenden (3S,1'R)-1-Benzyl-3-[(1 '-hydroxy)ethyl]-2-pyrrolidinons, um (3R,1'R)-1-Benzyl-3-[(1'-hydroxy)ethyl]pyrrolidin der Formel (3) herzustellen: 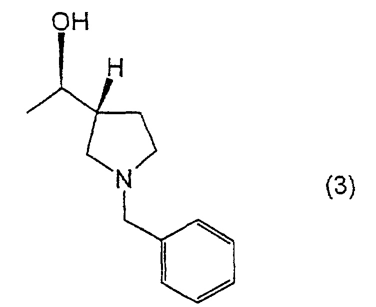 Mesylieren und/oder Tosylieren des resultierenden (3R,l'R)-1-Benzyl-3-[(1'-hydroxy)-ethyl]pyrrolidins, um (3R,1'R)-1-benzyl-3-[(1'-methansulfonyloxy)ethyl]pyrrolidin und/oder (3R,1'R)-1-Benzyl-3-[(1'-p-toluolsulfonyloxy)ethyl]pyrrolidin der Formel (4) herzustellen: 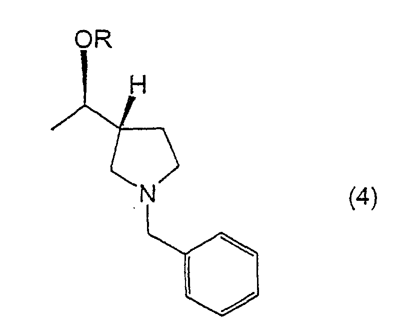 in welcher R eine Methansulfonylgruppe oder p-Toluolsulfonylgruppe bedeutet, und Methylaminieren des resultierenden (3R,1'R)-1-Benzyl-3-[(1'-methansulfonyloxy)-ethyl]pyrrolidins oder (3R,1'R)-1-Benzyl-3-[(1'-toluolsulfonyloxy)ethyl]pyrrolidins umfaßt.

2. Verfahren zur Herstellung von (3R,l'S)-1-Benzyl-3-[(1'-N-methylamino)ethyl]-pyrrolidin nach Anspruch 1, wobei das Bidentatphosphin ein Bidentatphosphin der folgenden allgemeinen Formel (6) ist: 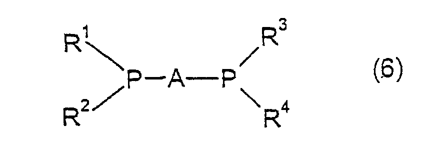 in welcher R¹, R², R³ und R⁴ gleich oder verschieden sein können und jeweils eine Phenylgruppe, Cyclohexylgruppe, Cyclopentylgruppe, einen C₁-C₄-Niederalkylrest, C₁-C₄-Niederalkoxyrest oder eine Halogen-substituierte Phenylgruppe bedeuten;
A durch die folgende allgemeine Formel (7) wiedergegeben wird: 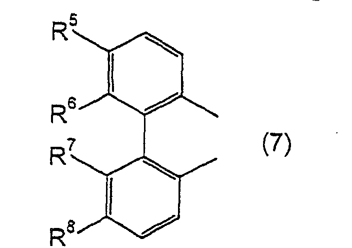 in welcher R⁵ und R⁸ jeweils ein Wasserstoffatom bedeuten, während R⁶ und R⁷ jeweils eine Methylgruppe bedeuten, oder R⁵ und R⁶ oder R⁷ und R⁸ unter Bildung einer Tetramethylengruppe vereinigt werden können, oder durch die folgende allgemeine Formel (8) wiedergegeben wird: 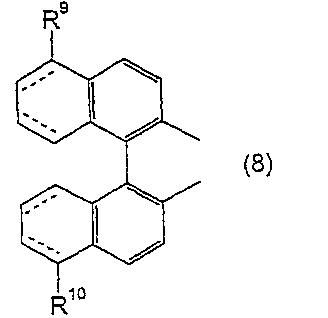 in welcher R⁹ und R¹⁰ gleich oder verschieden sein können und jeweils ein Wasserstoffatom, eine Aminogruppe, Acetylaminogruppe oder -SO₃M bedeuten, wobei M für ein Wasserstoffatom oder Alkalimetallatom steht, und die Kombination einer durchgezogenen und einer gestrichelten Linie entweder eine gesättigte Bindung oder eine Doppelbindung bedeutet.

3. Verfahren zur Herstellung von (3R,1'S)-1-Benzyl-3-[(1'-N-methylamino)ethyl]-pyrrolidin nach Anspruch 1 oder 2, wobei der aus Bidentatphosphin und Ruthenium gebildete Komplex ein Komplex ist, der durch die folgende allgemeine Formel (9) wiedergegeben wird:
RuₓH_{y}Cl_{z}(L)₂(S)ₚ (9)
in welcher L ein Bidentatphosphin bedeutet, das durch die folgende allgemeine Formel (6) wiedergegeben wird: 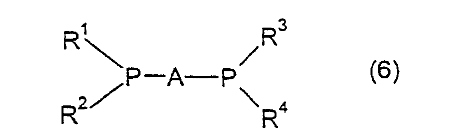 in welcher R¹, R², R³, R⁴ und A die gleichen Bedeutungen wie in Anspruch 2 definiert haben und S durch die folgende allgemeine Formel (10) wiedergegeben wird:
NR¹¹R¹²R¹³ (10)
in welcher R¹¹, R¹² und R¹³ gleich oder verschieden sein können und jeweils einen Niederalkylrest bedeuten, und wenn y 0 ist, x 2 ist, z 4 ist und p 1 ist; wenn y 1 ist, x 1 ist, z 1 ist und p 0 ist.

4. Verfahren zur Herstellung von (3R,1'S)-1-Benzyl-3-[(1'-N-methylamino)ethyl]-pyrrolidin nach Anspruch 1 oder 2, wobei der aus Bidentatphosphin und Ruthenium gebildete Komplex ein Komplex ist, der durch die folgende allgemeine Formel (11) wiedergegeben wird:
Ru(OCOR¹⁴)₂(L) (11)
in welcher R¹⁴ einen C₁-C₄-Niederalkylrest und eine Trifluormethylgruppe bedeutet, und L das gleiche Bidentatphosphin der Formel (6) in Anspruch 2 oder 3 darstellt.

5. Verfahren zur Herstellung von (3R,1'S)-1-Benzyl-3-[(1'-N-methylamino)ethyl]-pyrrolidin nach Anspruch 1 oder 2, wobei der aus Bidentatphosphin und Ruthenium gebildete Komplex ein Komplex ist, der durch die folgende allgemeine Formel (12) wiedergegeben wird:
RuX₂ (L) (12)
in welcher X ein Halogenatom bedeutet und L das gleiche Bidentatphosphin der Formel (6) wie in Anspruch 2 oder 3 definiert darstellt.

6. Verfahren zur Herstellung von (3R,1'S)-1-Benzyl-3-[(1'-N-methylamino)ethyl]-pyrrolidin nach Anspruch 1 oder 2, wobei der aus Bidentatphosphin und Ruthenium gebildete Komplex ein Komplex ist, der durch die folgende allgemeine Formel (13) wiedergegeben wird:
RuXₖJₘ(L)Qₙ (13)
in welcher X ein Halogenatom bedeutet und J Benzol, p-Cymol, C₁-C₄-Niederalkylbenzoat oder Acetonitril bedeutet, Q ein Halogenatom, ClO₄, PF₆ und BF₄ bedeutet, L das gleiche Bidentatphosphin der Formel (6) wie in Anspruch 2 oder 3 definiert darstellt; falls J Benzol, p-Cymol oder Niederalkylbenzoat ist, m 1 ist und n 1 ist, wenn k 1 ist; und falls J Acetonitril ist, m 1 ist und n 1 ist, wenn k 1 ist, und m 4 ist und n 2 ist, wenn k 0 ist.

7. Verfahren zur Herstellung von (3R,1'S)-1-Benzyl-3-[(1'-N-methylamino)ethyl]-pyrrolidin nach einem der Ansprüche 1 bis 6, wobei (3S,1'R)-1-Benzyl-3-[(1'-hydroxy)ethyl]-2-pyrrolidinon der Formel (2): 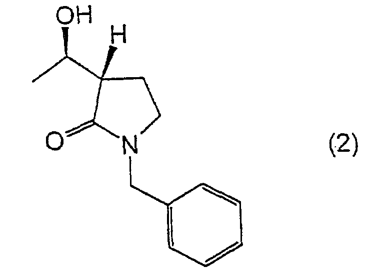 mit Lithiumaluminiumhydrid, Boran, Natriumborhydrid und einer Mineralsäure oder mit Natriumborhydrid und einer organischen Säure reduziert wird.

8. Verfahren zur Herstellung von (3R,1'S)-1-Benzyl-3-[(1'-N-methylamino)ethyl]-pyrrolidin nach einem der Ansprüche 1 bis 7, wobei (3S,1'R)-1-Benzyl-3-[(1'-hydroxy)ethyl]-2- pyrrolidin der Formel (3) : 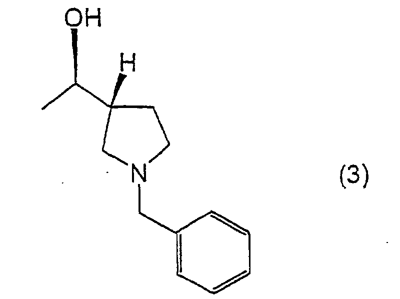 mit Methansulfonylchlorid oder p-Toluolsulfonylchlorid in Gegenwart einer Base umgesetzt wird.

9. Verfahren zur Herstellung von (3R,1'S)-1-Benzyl-3-[(1'-N-methylamino)ethyl]-pyrrolidin nach einem der Ansprüche 1 bis 8, wobei (3S,1'R)-1-Benzyl-3-[(1'-methansulfonyloxy)ethyl]-2-pyrrolidin und/oder (3R,1'R)-1-Benzyl-3-[1'-p-toluol-sulfonyloxy)ethyl]pyrrolidin der Formel (4): 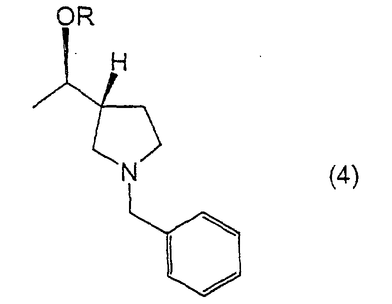 mit Methylamin in einem organischen Lösungsmittel umgesetzt werden.

## Revendications

1. Procédé pour la production de la (3R,1'S)-1-benzyl-3-[(1'-N-méthylamino)éthyl]pyrrolidine représentée par la formule (5) : 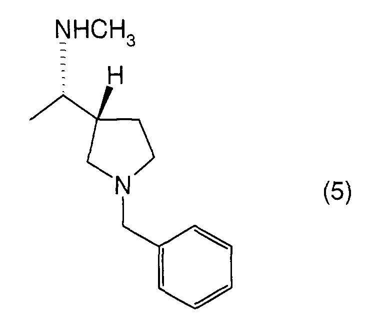 **caractérisé en ce que** ledit procédé comprend l'hydrogénation asymétrique de la 3-acétyl-1-benzyl-2-pyrrolidinone représentée par la formule (1): 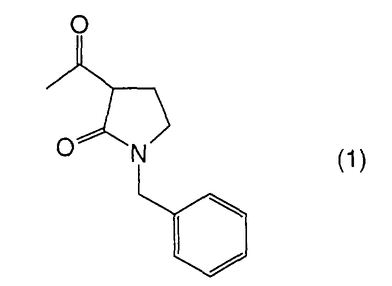 par utilisation d'un complexe formé à partir d'une phosphine bidentée et de ruthénium en tant que catalyseur pour produire la (3S,1'R)-1-benzyl-3-[(1'-hydroxy)éthyl]-2-pyrrolidinone représentée par la formule (2) : 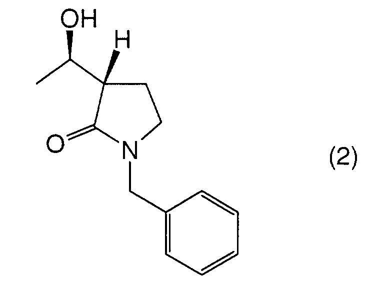 la réduction de la (3S,1'R)-1-benzyl-3-[(1'-hydroxy)éthyl]-2-pyrrolidinone formée pour produire la (3R,1'R)-1-benzyl-3-[(1'-hydroxy)éthyl]pyrrolidine représentée par la formule (3): 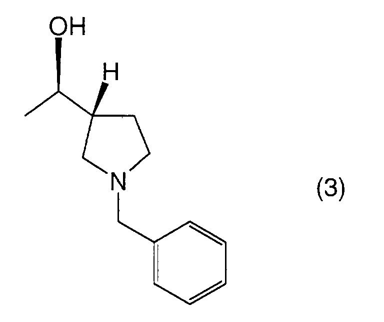 la mésylation et/ou la tosylation de la (3R,1'R)-1-benzyl-3-[(1'-hydroxy)éthyl]pyrrolidine obtenue pour produire la (3R,1'R)-1-benzyl-3-[(1'-méthanesulfonyloxy)éthyl]pyrrolidine et/ou la (3R,1'R)-1-benzyl-3-[(1'-p-toluènesulfonyloxy)éthyl]pyrrolidine représentée par la formule (4) : 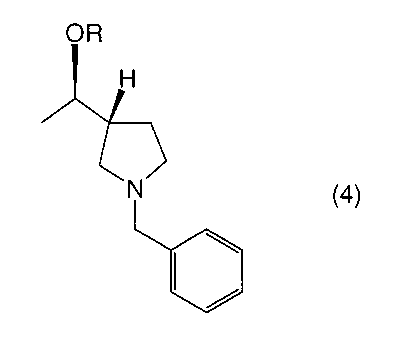 où R représente un groupe méthanesulfonyle ou un groupe p-toluènesulfonyle, et la méthylamination de la (3R,1'R)-1-benzyl-3-[(1'-méthanesulfonyloxy)éthyl]pyrrolidine ou de la (3R,1'R)-1-benzyl-3-[(1'-toluènesulfonyloxy)éthyl]pyrrolidine obtenue.

2. Procédé pour la production de la (3R,1'S)-1-benzyl-3-[(1'-N-méthylamino)éthyl]pyrrolidine selon la revendication 1, dans laquelle la phosphine bidentée est une phosphine bidentée représentée par la formule générale suivante (6): 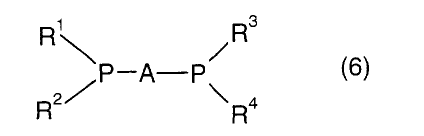 dans laquelle R¹, R², R³ et R⁴ peuvent être identiques ou différents et représentent chacun un groupe phényle, un groupe cyclohexyle, un groupe cyclopentyle, un groupe alkyle inférieur en C₁ à C₄, un groupe alcoxy inférieur en C₁ à C₄ ou un groupe phényle substitué par un halogène ;
A est représenté par la formule générale suivante (7) : 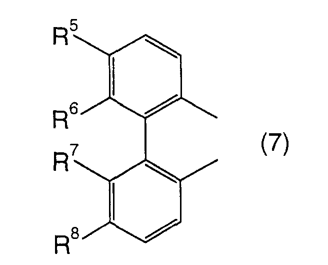 où R⁵ et R⁸ représentent chacun un atome d'hydrogène tandis que R⁶ et R⁷ représentent chacun un groupe méthyle, ou R⁵ et R⁶ ou R⁷ et R⁸ peuvent être combinés pour former un groupe tétraméthylène, ou est représenté par la formule générale suivante (8) : 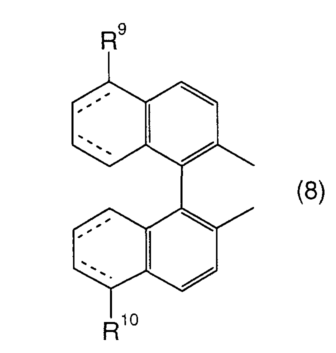 où R⁹ et R¹⁰ peuvent être identiques ou différents et représentent chacun un atome d'hydrogène, un groupe amino, un groupe acétylamino ou -SO₃M où M représente un atome d'hydrogène ou un atome de métal alcalin, la combinaison d'une ligne continue et d'une ligne en tirets représentant une liaison saturée ou une double liaison.

3. Procédé pour la production de la (3R,1'S)-1-benzyl-3-[(1'-N-méthylamino)éthyl]pyrrolidine selon la revendication 1 ou 2, dans lequel le complexe formé entre la phosphine bidentée et le ruthénium est un complexe représenté par la formule générale suivante(9):
RuₓH_{y}Cl_{z}(L)₂(S)ₚ (9)
où L est une phosphine bidentée représentée par la formule générale suivante (6) : 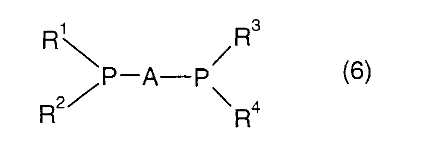 dans laquelle R¹, R², R³, R⁴ et A ont les mêmes définitions que dans la revendication 2 et S est représenté par la formule générale suivante (10):
NR¹¹R¹²R¹³ (10)
dans laquelle R¹¹, R¹² et R¹³ peuvent être identiques ou différents et représentent chacun un groupe alkyle inférieur, et si y est 0, x est 2, z est 4 et p est 1 ; si y est 1, x est 1, z est 1 et p est 0.

4. Procédé pour la production de la (3R,1'S)-1-benzyl-3-[(1'-N-méthylamino)éthyl]pyrrolidine selon la revendication 1 ou 2, dans lequel le complexe formé entre la phosphine bidentée et le ruthénium est un complexe représenté par la formule générale suivante (11):
Ru(OCOR¹⁴)₂(L) (11)
où R¹⁴ représente un groupe alkyle inférieur en C₁ à C₄ et un groupe trifluorométhyle et L représente la même phosphine bidentée de formule (6) que dans la revendication 2 ou 3.

5. Procédé pour la production de la (3R,1'S)-1-benzyl-3-[(1'-N-méthylamino)éthyl]pyrrolidine selon la revendication 1 ou 2, dans lequel le complexe formé entre la phosphine bidentée et le ruthénium est un complexe représenté par la formule générale suivante (12) :
RuX₂(L) (12)
où X représente un atome d'halogène et L représente la même phosphine bidentée de formule (6) que dans la revendication 2 ou 3.

6. Procédé pour la production de la (3R,1'S)-1-benzyl-3-[(1'-N-méthylamino)éthyl]pyrrolidine selon la revendication 1 ou 2, dans lequel le complexe formé entre la phosphine bidentée et le ruthénium est un complexe représenté par la formule générale suivante (13) :
RuXₖJₘ(L)Qₙ (13)
où X représente un atome d'halogène et J représente le benzène, le p-cymène, un benzoate d'alkyle inférieur en C₁ à C₄ ou l'acétonitrile, Q représente un atome d'halogène, ClO₄, PF₆ et BF₄, L représente la même phosphine bidentée de formule (6) que dans la revendication 2 ou 3 ; dans le cas où J est le benzène, le p-cymène ou un benzoate d'alkyle inférieur, m est 1 et n est 1 si k est 1 ; et dans le cas où J est l'acétonitrile, m est 1 et n est 1 si k est 1 et m est 4 et n est 2 si k est 0.

7. Procédé pour la production de la (3R,1'S)-1-benzyl-3-[(1'-N-méthylamino)éthyl]pyrrolidine selon l'une quelconque des revendications 1 à 6, dans lequel la (3S,1'R)-1-benzyl-3-[(1'-hydroxy)éthyl]-2-pyrrolidinone représentée par la formule (2) : 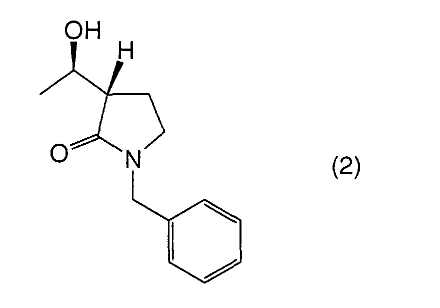 est réduite par de l'hydrure de lithium aluminium, un borane, du borohydrure de sodium et un acide minéral ou par le borohydrure de sodium et un acide organique.

8. Procédé pour la production de la (3R,1'S)-1-benzyl-3-[(1'-N-méthylamino)éthyl]pyrrolidine selon l'une quelconque des revendications 1 à 7, dans lequel on fait réagir la (3S,1'R)-1-benzyl-3-[(1'-hydroxy)éthyl]-2-pyrrolidine représentée par la formule (3): 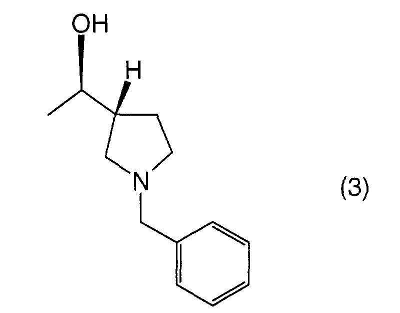 avec du chlorure de méthanesulfonyle ou du chlorure de p-toluènesulfonyle en présence d'une base.

9. Procédé pour la production de la (3R,1'S)-1-benzyl-3-[(1'-N-méthylamino)éthyl]pyrrolidine selon l'une quelconque des revendications 1 à 8, dans lequel on fait réagir la (3S,1'R)-1-benzyl-3-[(1'-méthanesulfonyloxy)éthyl]-2-pyrrolidine et/ou la (3R,1'R)-1-benzyl-3-[1'-p-toluènesulfonyl-oxy)éthyl]pyrrolidine représentées par la formule (4): 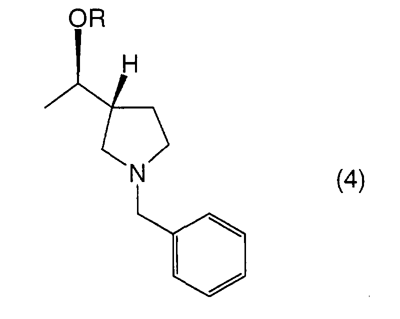 avec la méthylamine dans un solvant organique.
